(19)

Europäisches Patentamt
European Patent Office
Office européen des brevets

(11) **EP 4 509 817 A1**

(12) **EUROPEAN PATENT APPLICATION**
published in accordance with Art. 153(4) EPC

(43) Date of publication:
**19.02.2025  Bulletin 2025/08**

(21) Application number: **23788646.0**

(22) Date of filing: **14.04.2023**

(51) International Patent Classification (IPC):
*G01N 21/15* [(2006.01)]     *B01L 3/00* [(2006.01)]
*G06T 7/70* [(2017.01)]      *G06T 7/11* [(2017.01)]
*G08B 21/18* [(2006.01)]     *G06Q 50/10* [(2012.01)]

(52) Cooperative Patent Classification (CPC):
**B01L 3/00; G01N 21/15; G06Q 50/10; G06T 7/11;
G06T 7/70; G08B 21/18**

(86) International application number:
**PCT/KR2023/005092**

(87) International publication number:
**WO 2023/200297 (19.10.2023 Gazette 2023/42)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **15.04.2022  KR 20220046896**

(71) Applicants:
• **Seegene, Inc.**
  **Seoul 05548 (KR)**
• **Seegene Medical Foundation**
  **Seoul 04805 (KR)**

(72) Inventors:
• **SUNG, Nack Moon**
  **Yongin-si Gyeonggi-do 16899 (KR)**
• **PARK, Bong Gyun**
  **Seoul 06069 (KR)**
• **JUNG, Hwa Young**
  **Seoul 02810 (KR)**
• **LIM, Jeong Min**
  **Seoul 05241 (KR)**

(74) Representative: **Viering, Jentschura & Partner
mbB
Patent- und Rechtsanwälte
Am Brauhaus 8
01099 Dresden (DE)**

(54) **METHOD FOR ESTIMATING WHETHER PLATE IS CONTAMINATED, AND COMPUTER DEVICE**

(57)     According to an embodiment, disclosed is a method for assuming a contamination of a plate used for a nucleic acid amplification test, performed by a computer device. The method may include: obtaining a detection result of a target analyte of a plurality of reaction wells in the plate; obtaining a position data of positive reaction wells in the plate from the detection result; and assuming the contamination of the plate by at least partially using the obtained position data.

*FIG.2*

**EP 4 509 817 A1**

**Description**

[Technical Field]

The present invention relates to a method and a computer device for assuming the presence or absence of contamination of a plate.

[Background Art]

In order to manage the quality of the molecular diagnostic test results, it is necessary to check whether contamination has occurred during the molecular diagnostic test process. There are various causes that cause this contamination. This includes, for example, cross-contamination which occurs between the reaction wells. Such mutual contamination may occur in the process of dispensing the sample into the reaction well.

There are various methods for identifying the aforementioned contamination. For example, this includes performing a retest on the corresponding sample to compare whether there is a difference in the test results. However, in this case, considerable time and cost are required for retest. In addition, the waiting time of the examinee will be increased until the test result is confirmed. Alternatively, if such retest is skipped, there is a problem that incorrect test result due to contamination may result in disadvantages for the examinee or delays in prompt response to the examinee. As a result, significant personal and/or social costs may be incurred.

[Disclosure]

[Technical Problem]

A problem to be solved by an embodiment of the present invention is to provide a method capable of assuming whether contamination has occurred in any reaction well in the plate.

However, problems to be solved by the present disclosure are not limited to the foregoing, and other unmentioned objects would be apparent to one of ordinary skill in the art from the following description.

[Technical Solution]

Disclosed is a method for assuming a contamination of a plate used for a nucleic acid amplification test, performed by a computer device according to an embodiment of the present disclosure. The method may include: obtaining a detection result of a target analyte of a plurality of reaction wells in the plate; obtaining a position data of positive reaction wells in the plate from the detection result; and assuming the contamination of the plate by at least partially using the obtained position data.

In an embodiment, wherein the contamination may include a false-positive occurrence in at least some of the positive reaction wells due to one or more contamination sources, and wherein the one or more contamination sources may include at least one selected from the group consisting of: a first contamination source caused by a nucleic acid molecule extracted from a pathogen in a positive reaction well being propagated into the air; a second contamination source caused by the nucleic acid molecule of a pipette in contact with the positive reaction well; a third contamination source caused by a poor sealing of the reaction well, and a combination thereof.

In an embodiment, wherein the assuming the contamination may include: determining at least one of (a) a distance between the positive reaction wells, (b) a density of the positive reaction wells, and (c) an area or a shape of a figure defined according to a line connecting the positive reaction wells based on the obtained position data; and assuming the contamination by using at least one of: the distance; the density; and the area or the shape of the figure.

In an embodiment, the method may further include obtaining an image area for the position of the plurality of reaction wells based on the position data, wherein the assuming the contamination may include: detecting a count of positive reaction wells included in each sliding window while moving a sliding window of a preset size by a preset unit on the image area; and calculating an assumed value for the contamination based on the count of positive reaction wells included in each sliding window.

In an embodiment, wherein the detecting the contamination may be performed by using the sliding window of the preset size and then performed by using each of sliding windows of a size different from the preset size, and the assumed value may be calculated based on the count of positive reaction wells included in each sliding window detected by using the sliding window of the preset size and the sliding window of the size different from the preset size.

In an embodiment, wherein the moving the sliding window may include: moving the sliding window on the image area by a preset unit smaller than the preset size; or moving the sliding window on the image area so that a part of the positive reaction wells included in the sliding window before moving is included in the sliding window after moving.

In an embodiment, wherein the calculating the assumed value may include: detecting a frequency for each count of positive reaction wells in the sliding window; and calculating a degree to which the positive reaction wells are adjacent to each other in the plate, as the assumed value, by using each count of the positive reaction wells in the sliding window, the size, and the frequency.

In an embodiment, wherein the assuming the contamination may include assuming a contamination in each of the positive reaction wells by using at least a part of the obtained position data.

In an embodiment, wherein the assuming the contamination may include assuming the contamination of the plate by at least partially using the assumed result of the contamination in each of the positive reaction wells.

In an embodiment, wherein the assuming the contamination in each of the positive reaction wells may include: determining a distance between the positive reaction wells by using the obtained position data; and calculating each of a first assumed values indicating a contamination possibility in each of the positive reaction wells by using the distance between the positive reaction wells.

In an embodiment, wherein the distance between the positive reaction wells may be calculated based on a Manhattan distance measurement method.

In an embodiment, wherein the distance may be a distance between each positive reaction well and other positive reaction wells, and wherein the each of the first assumed values may be determined by at least partially using a result of summing reciprocals of the distance between each positive reaction well and other positive reaction wells.

In an embodiment, wherein the assuming the contamination may further include calculating a second assumed value indicating a contamination possibility of the plate by using a result of summing the first assumed values.

In an embodiment, the method may further include displaying a result of the assuming the contamination.

In an embodiment, the method may further include outputting a warning message indicating that at least in part of the positive reaction wells are likely to be positive due to contamination, when an assumed value indicating the contamination possibility of the plate is not less than a predetermined threshold value.

In an embodiment, wherein the warning message may further include a position data of a positive reaction well assumed to be contaminated among the positive reaction wells, and wherein the positive reaction well assumed to be contaminated may be determined by using an assumed value indicating a contamination probability in each of the positive reaction wells.

In an embodiment, wherein the threshold value may be determined based on an interquartile range (IQR), a third quartile (Q3), and preset weight which are determined from a dataset for the assumed values of a plurality of plates.

In an embodiment, an assumed value indicating a contamination possibility of the plate may be calculated for each of a plurality of plates, and wherein the method may further include aligning and displaying the assumed value of each of the plurality of plates.

In an embodiment, the method may further include providing an assumption information about a contamination pattern of the positive reaction wells by using the position data of the positive reaction wells.

In an embodiment, the method may further include: obtaining a re-detection result of the target analyte in the positive reaction wells; and updating an equation used to assume the contamination and/or a threshold value which is a criterion for assuming the contamination, by using a comparison result between the re-detection result and the detection result.

In an embodiment, when any one positive reaction well among the positive reaction wells is determined to be positive in the detection result but is determined to be negative in the re-detection result, the updating may be performed based on a difference of assumed values of the contamination calculated from each of the re-detection result and the detection result.

In an embodiment, wherein the assuming the contamination may include assuming the contamination of the plate by additionally using a positive-negative determination basis obtained from the detection result.

In an embodiment, wherein the positive-negative determination basis may include at least one selected from the group consisting of (a) a cycle value indicating a time or reaction number for an intensity of a signal value to reach a predetermined threshold value in a set of signal values obtained from the detection result, (b) a signal value at a specific cycle in the set of the signal values, and (c) a combination thereof.

In an embodiment, wherein the assuming the contamination may include: determining at least one of (a) a distance between the positive reaction wells, (b) a density of the positive reaction wells, and (c) an area or a shape of a figure defined according to a line connecting the positive reaction wells based on the obtained position data; and calculating an assumed value indicating a contamination possibility of the plate, by using (i) at least one of the distance, the density, the area or the shape of the figure, and (ii) the positive-negative determination basis between the positive reaction wells.

In an embodiment, wherein the assumed value may be calculated based on a result of applying each weight to reciprocals of each distance between each positive reaction well and other positive reaction wells and summing thereof, and wherein the weight may be determined based on the positive-negative determination basis between each positive reaction well and other positive reaction wells.

Disclosed is a computer program stored on a computer-readable recording medium according to an embodiment of the present disclosure. The computer program may be programmed to perform each step included in the method according to the present disclosure.

Disclosed is a computer-readable recording medium on which a computer program is stored according to an embodiment of the present disclosure. The computer program may be programmed to perform each step in the method according to the present disclosure.

Disclosed is a computer device according to an embodiment of the present disclosure. The computer device may include: a memory including at least one instruction; and a processor. As the at least one instruction is executed by the processor: a detection result of a target analyte of a plurality of reaction wells in the plate is obtained; a position data of positive reaction wells in the plate is obtained from the detection result; and the contamination of the plate is assumed by at least partially using the obtained position data.

Disclosed is a method for managing quality of a laboratory space performed by a computer device according to an embodiment of the present disclosure. The method may include: obtaining a detection result of a target analyte of a plurality of reaction wells in the plate; obtaining a position data of positive reaction wells in the plate from the detection result; and assuming the contamination of the plate by at least in part using the obtained position data; and providing a quality control level of the laboratory space in which a detection process using the plate is performed, based at least in part on the contamination and a count of the positive reaction wells.

In an embodiment, wherein the providing the quality control level of the laboratory space may include: outputting a first message indicating that a positive state for the quality control level is assumed, when a ratio of the count of positive reaction wells to an assumed value indicating a contamination possibility of the plate is not less than a first value; and outputting a second message indicating that a negative state for the quality control level is assumed, when the ratio of the count of positive reaction wells to the assumed value is not larger than a second value, which is less than the first value.

In an embodiment, wherein the first message may further include a message recommending to skip performing a re-detection process for some plates in which the ratio of the count of positive reaction wells to the assumed value is not less than the first value, among a plurality of plates.

In an embodiment, wherein the second message may further include a message recommending at least one of (a) cleaning the laboratory space, (b) replacing a pipette, and (c) re-performing the detection process for some plates in which the ratio of the count of positive reaction wells to the assumed value is not larger than the second value, among the plurality of plates.

[Description of Drawings]

FIG. 1 is a block diagram illustrating a contamination assumption system according to an embodiment of the present disclosure.

FIG. 2 is a block diagram schematically illustrating a configuration of the computer device according to an embodiment.

FIG. 3 is a diagram illustrating modularized software implemented by the computer device illustrated in FIG. 2.

FIG. 4 is a flowchart illustrating a method, performed by the computer device, for assuming a contamination of a plate used for a nucleic acid amplification test, according to an embodiment.

FIG. 5 is a diagram illustrating the position data of the positive reaction wells in the plate according to an embodiment.

FIG. 6 is a diagram for explaining the operation of assuming contamination by using the position data of positive reaction wells, by the contamination assumption unit according to the first embodiment.

FIG. 7 is a diagram for explaining the operation of assuming contamination by using the density of positive reaction wells, by the contamination assumption unit according to the second embodiment.

FIG. 8 is a diagram for explaining the operation of assuming contamination by using the area or shape of the figure defined according to the line connecting the positive reaction wells, by the contamination assumption unit according to the third embodiment.

FIG. 9 is a flowchart illustrating a method for assuming contamination by using the sliding window, by the contamination assumption unit according to the fifth embodiment.

FIG. 10 to FIG. 11 are diagrams for explaining the operation of assuming contamination by using the first sliding window and the second sliding window, by the contamination assumption unit according to the second embodiment.

FIG. 12 is a graph for explaining the operation of assuming contamination by comparing the assumed value with the threshold value, by the contamination assumption unit according to the sixth embodiment.

FIG. 13 is a graph for explaining the operation of setting the threshold value by using a box plot-based statistical distribution, by the contamination assumption unit according to the sixth embodiment.

FIG. 14 is a flowchart illustrating a method for assuming contamination of each of the plurality of reaction wells and assuming contamination of the plate based on the assumption result, by the contamination assumption unit according to the seventh embodiment.

FIG. 15 to FIG. 17 are diagrams for explaining the operation of calculating the plurality of first assumed values indicating the contamination possibility in each of the plurality of positive reaction wells, by the contamination assumption unit according to the seventh embodiment.

FIG. 18 is a diagram for explaining the operation of providing plate identifiers in order from highest to lowest assumed values among the plurality of plates, by the contamination warning unit according to an embodiment.

FIG. 19 is a diagram for explaining the operation of providing assumption information about the pattern of positive reaction wells based on the position data of the positive reaction wells, by the contamination warning unit according to an embodiment.

FIG. 20 is a flowchart illustrating a method for managing quality of a laboratory space performed by a computer device according to an embodiment.

FIG. 21 is a diagram for explaining the operation of providing quality control level of the laboratory space based on the assumed value of the contamination and the count of the positive reaction wells, by the quality management unit according to an embodiment.

[Mode for Invention]

Advantages and features of the present invention, and methods for achieving them, will become clear with reference to the embodiments described below in detail in conjunction with the accompanying drawings. However, the present invention is not limited to the embodiments disclosed below, but may be implemented in various different forms, and only the present embodiments make the disclosure of the present invention complete, and common knowledge in the art to which the present invention belongs It is provided to fully inform the holder of the scope of the invention, and the present invention is only defined by the scope of the claims.

In the description of the present invention, if it is determined that a detailed description of a related known function or configuration may unnecessarily obscure the subject matter of the present invention, the detailed description will be omitted. In addition, terms to be described later are terms defined in consideration of functions in the present invention, which may vary according to the intention or custom of a user or operator. Therefore, the definition should be made based on the contents throughout this specification.

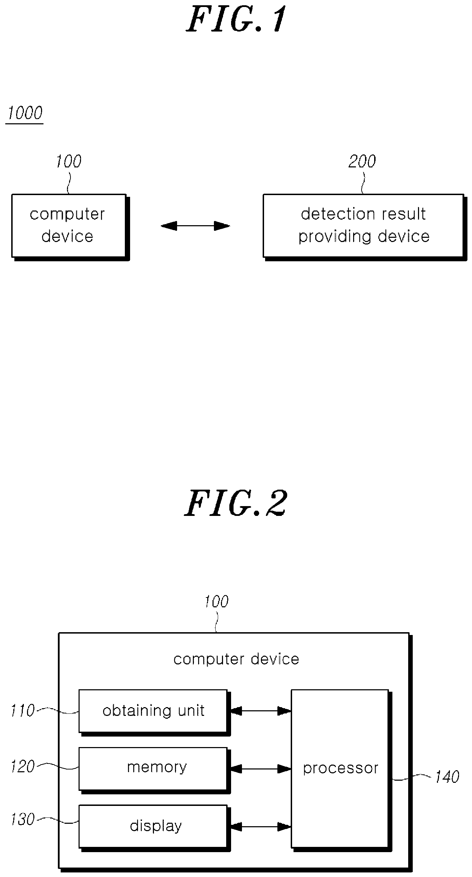

Prior to describing FIG. 1, terms used herein are described.

As used herein, the term "detection process" refers to a process for a signal generation reaction, and may be interpreted as a concept including a process required for preparation of a signal generation reaction according to an embodiment. As used herein, the term "signal generation reaction" refers to a reaction that generates a signal dependent on the properties of a target analyte in a sample, e.g., activity, amount, or presence (or absence), specifically generates a signal dependent on the presence (or absence). This signal generation reaction includes a biological reaction and a chemical reaction. Among them, the biological reaction includes a genetic analysis process such as polymerase chain reaction (PCR), real-time PCR, real-time isothermal amplification reaction, and microarray analysis, an immunological analysis process, and a bacterial growth analysis. In addition, the chemical reaction includes a process of analyzing the production, change, or destruction of a chemical substance. According to an embodiment, the signal generation reaction may be the genetic analysis process, or may be a nucleic acid amplification reaction such as the PCR or the isothermal amplification reaction, an enzymatic reaction, or microbial growth. In the amplification reaction, amplification of a target analyte (e.g., a nucleic acid molecule) may or may not be accompanied. More specifically, the amplification reaction may mean an amplification reaction of a signal accompanied by amplification of the target analyte.

According to an embodiment, the signal generation reaction may include a nucleic acid amplification reaction amplifying a target analyte (e.g., viral or bacterial nucleic acid). As a representative example of the nucleic acid amplification reaction, in the PCR, a repeated cycle process of denaturation of double-stranded DNA, annealing of an oligonucleotide primer to a DNA template, and primer extension by DNA polymerase is performed (Mullis et al., U.S. Pat. Nos. 4, 683, 195, 4, 683, 202, and 4, 800, 159; Saiki et al., Science 230: 1350-1354 (1985)). As other methods for the amplifying the nucleic acid, various methods such as Ligase Chain Reaction (LCR), Strand Displacement Amplification (SDA), Nucleic Acid Sequence-Based Amplification (NASBA), Transcription Mediated Amplification (TMA), Recombinase Polymerase Amplification (RPA), Loop-mediated isothermal amplification (LAMP), and Rolling-Circle Amplification (RCA) have been proposed.

In addition, the term "plate" refers to a reference unit in which a detection process is performed, for example, a basic unit in which data generated after an amplification reaction is stored. For example, the different plates may be plates on which the detection process is performed at different times by using the same detection device (e.g., a PCR device), or plates on which nucleic acid amplification reactions are performed by different detection devices at the same time.

A plate includes a plurality of reaction wells, and may include, for example, N x M reaction wells. Typically, the plate contains 12 x 8 or 8 x 12 reaction wells. The reaction well of the plate may be integral with the plate or in the form of a detachable tube. The plate may have a rectangular shape. However, the plate includes one or more reaction wells and may be implemented in various shapes such as a circle, a ladder, a rhombus, and the like in addition to the rectangle.

A sample(s) to be analyzed and a signal generation composition(s) required for the signal generation reaction may be accommodated in the reaction well of the plate. Here, the signal generation composition means a reagent(s) used in the signal generation reaction, and may include, for example, a primer, a probe, an enzyme, a buffer, dNTP, or the like.

In addition, the term "target analyte" may refer to various substances (e.g., biological substances and non-biological substances). Specifically, the target analyte may include a biological substance, more specifically at least one of nucleic acid molecules (e.g., DNA and RNA), proteins, peptides, carbohydrates, lipids, amino acids, biological compounds, hormones, antibodies, antigens, metabolites, and cells.

In addition, the term "sample" may include a biological sample (e.g., cells, tissues, and body fluids) and an abiotic sample (e.g., food, water, and soil). The biological sample may include at least one of, for example, virus, bacteria, tissue, cells, blood (including whole blood, plasma, and serum), lymph, bone marrow, saliva, sputum, swab, aspiration, milk, urine, stool, eye fluid, semen, brain extract, spinal fluid, joint fluid, thymus fluid, bronchial lavage fluid, ascites, and amniotic fluid. These samples may or may not include the target analyte described above.

On the other hand, when the target analyte described above is a nucleic acid molecule or includes a nucleic acid molecule, a nucleic acid extraction process known in the art may be performed on the sample assumed to include the target analyte (see Sambrook, J. et al., Molecular Cloning. A Laboratory Manual, 3rd ed. Cold Spring Harbor Press(2001)). The nucleic acid extraction process may vary depending on the type of sample. In addition, when the extracted nucleic acid is RNA, a reverse transcription process for synthesizing cDNA may be additionally performed (see Sambrook, J. et al., Molecular Cloning. A Laboratory Manual, 3rd ed. Cold Spring Harbor Press(2001)).

In addition, the term "detection result" may refer to a result obtained according to a detection process, for example, result data such as whether a target analyte has been detected for each reaction well based on a signal generation reaction occurring in each of a plurality of reaction wells in the plate, a signal value or a measurement value used to analyze the target analyte, and the like. According to an embodiment, the detection result may be data obtained by processing the result data obtained according to the detection process.

Hereinafter, various embodiments of the present invention will be described with reference to the drawings.

FIG. 1 is a block diagram illustrating a contamination assumption system 1000 according to an embodiment of the present disclosure.

Referring to FIG. 1, the contamination assumption system 1000 may include a computer device 100 and a detection result providing device 200. The computer device 100 may assume a contamination of a plate. In an embodiment, the computer device 100 may be implemented to analyze a detection result of a target analyte using a plate as a reference unit and assume (i) whether contamination has occurred in one or more of a plurality of reaction wells in the plate and/or (ii) a possibility of contamination occurrence.

In an embodiment, the computer device 100 may be implemented as a computer operating through a computer program for realizing functions described herein. For example, the computer device 100 may be provided in a laboratory space in which a detection process of a target analyte is performed, or may be implemented as a computer used by a user in the laboratory space. As another example, the computer device 100 may be implemented as a server that manages a plurality of laboratory spaces. As another example, the computer device 100 may be included in a detection device that is provided in a laboratory space and performs a detection process using a plate as a reference unit.

The detection result providing device 200 may obtain a detection result for a detection process performed using a plate as a reference unit. In an embodiment, the detection result providing device 200 may be implemented as a nucleic acid amplification device that performs the detection process. In another embodiment, the detection result providing device 200 may be implemented as a computer or the like in which a viewer program for analyzing a nucleic acid amplification result generated by the nucleic acid amplification device and providing positive/negative information is executed. In another embodiment, the detection result providing device 200 may be implemented as a server (e.g., a medical institution server) that stores and manages a plurality of detection results of a plurality of examinees obtained in advance.

As illustrated in FIG. 1, the computer device 100 may be connected to the detection result providing device 200. For example, the computer device 100 may be connected to the detection result providing device 200 through a network or electrically. Here, the network may be configured through various communication networks such as wired and wireless networks, and for example, may be configured as various communication networks such as a Local Area Network (LAN), a Metropolitan Area Network (MAN), a Wide Area Network (WAN), and the like.

In an embodiment, the computer device 100 may obtain the detection results of the target analyte of the plurality of reaction wells in the plate from the detection result providing device 200. For example, the computer device 100 may receive a detection result including a signal or a measurement value (e.g., an amplification curve) indicating a nucleic acid amplification result from the detection result providing device 200 implemented as the nucleic acid amplification device. As another example, the computer device 100 may receive the detection result including positive/negative information for each reaction well in the plate from the detection result providing device 200 implemented as a computer in which the viewer program is executed. In another embodiment, the computer device 100 may directly obtain the detection result by performing the detection process of the target analyte using the plate as the reference unit.

Meanwhile, the contamination assumption system 1000 may further include other components in addition to the components shown in FIG. 1. Alternatively, one of the components illustrated in FIG. 1 may be omitted.

In an embodiment, the following functions performed by the computer device 100 may be implemented in the detection result providing device 200. For example, a function of assuming the following contamination may be mounted on the nucleic acid amplification device, and for another example, may be implemented as software in the computer on which the viewer program is executed.

FIG. 2 is a block diagram schematically illustrating a configuration of the computer device 100 according to an embodiment.

Referring to FIG. 2, the computer device 100 may include an obtaining unit 110, a memory 120, a display 130, and a processor 140.

The obtaining unit 110 may obtain a detection result of a target analyte of a plurality of reaction wells in a plate. In an embodiment, the detection result may include whether the target analyte to be analyzed is detected in a sample included in each of the plurality of reaction wells in the plate, according to the detection process performed using the plate as the reference unit.

In an embodiment, the obtaining unit 110 may include a transceiver (not shown), and may receive the detection result of the target analyte from the detection result providing device 200 by using the transceiver. In another embodiment, the obtaining unit 110 may read the detection result of the target analyte pre-stored in a storage device from the corresponding device electrically connected thereto (e.g., a universal serial bus (USB), the memory 120, or the like), and is not limited to the above-described embodiment.

The memory 120 may store at least one instruction or data to be executed by the processor 140 to be described below. In an embodiment, the memory 120 may include at least one of a read only memory (ROM) and a random access memory (RAM).

The display 130 may display an image, a graph, a table, a text, or the like obtained as at least one instruction is executed by the processor 140. For example, the display 130 may display a plate identifier, a reaction well identifier, status information (e.g., positive/negative information) for each reaction well, an assumed value indicating whether contamination occurs, and a contamination possibility, as an image or text.

The display 130 may comprehensively mean a data output device for displaying data. For example, the display 130 may be implemented as a liquid crystal display, a thin film transistor-liquid crystal display, an organic light-emitting diode, a flexible display, a three-dimensional (3D) display, or the like.

The processor 140 may control the overall operation of the computer device 100, and may perform a series of operations for assuming contamination of a plate. The processor 140 may refer to a central processing unit (CPU), a graphics processing unit (GPU), a micro controller unit (MCU), or a dedicated processor on which methods according to embodiments are performed.

In addition, it may be understood by those of ordinary skill in the art that other general-purpose components other than the components illustrated in FIG. 2 may be further included in the computer device 100. For example, the computer device 100 may further include a bus line used for transmission of data between components, various input/output devices, and interfaces therefor.

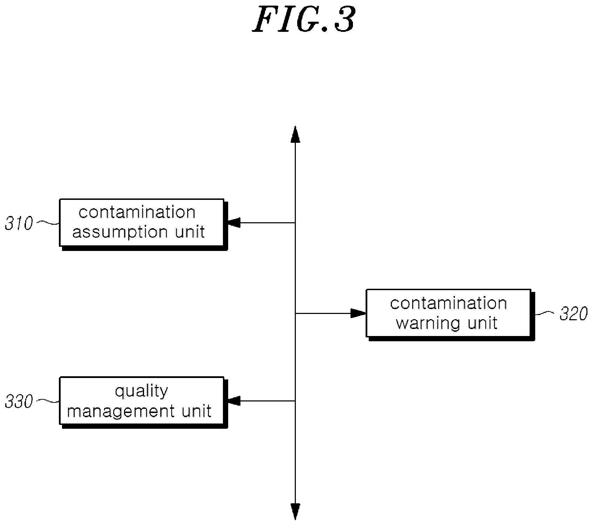

The processor 140 may provide various functions by executing at least one instruction stored in the memory 120. Various functions provided by the processor 140 will be described with reference to FIG. 3.

FIG. 3 is a diagram illustrating modularized software implemented by the computer device 100 illustrated in FIG. 2. Referring to FIG. 3, software implemented as the processor 140, which is hardware, executes at least one instruction stored in the memory 120 may be modularized into at least one of a contamination assumption unit 310, a contamination warning unit 320, and a quality management unit 330. For example, each of the contamination assumption unit 310, the contamination warning unit 320, and the quality management unit 330 may be implemented as a computer program, and instructions and data for each of these executions may be stored in the memory 120 and executed by the processor 140.

In an embodiment, the contamination assumption unit 310 may refer to software programmed to assume the contamination of the plate, the contamination warning unit 320 may refer to software programmed to warn of a contamination possibility based on the assumed contamination, and the quality management unit 330 may refer to software programmed to provide a quality control level of a laboratory space in which the detection process using the plate is performed, but is not limited thereto.

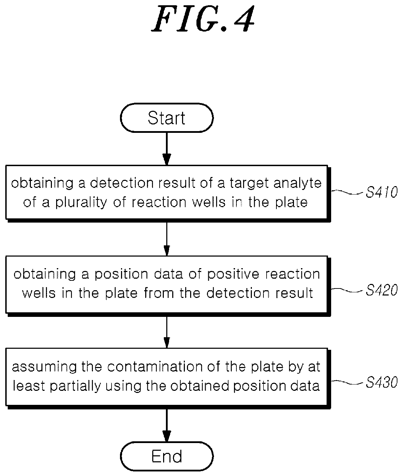

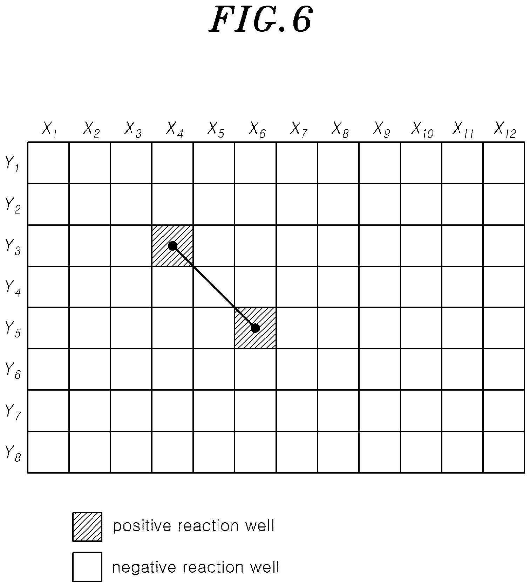

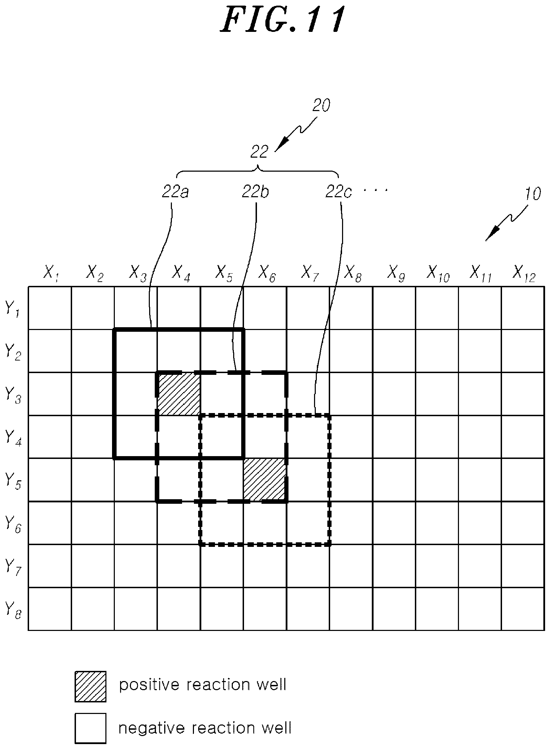

Hereinafter, the overall operation of the computer device 100 will be schematically described with reference to FIG. 4 to FIG. 5, and then the functions and/or operations of each of the contamination assumption unit 310, the contamination warning unit 320, and the quality management unit 330 will be described in detail with reference to FIG. 6 to FIG. 21.

FIG. 4 is a flowchart illustrating a method, performed by the computer device 100, for assuming a contamination of a plate used for a nucleic acid amplification test, according to an embodiment. Here, the nucleic acid amplification test may be used in various technical fields for detecting a target analyte using the nucleic acid amplification reaction. In an embodiment, the nucleic acid amplification test may be performed on various objects such as soil, water, and plants, and by detecting whether nucleic acid molecules extracted from pathogens (e.g., viruses) are included in such objects, it may be tested for soil contamination, water contamination, or pathogen infection of animals and/or plants. The embodiments described throughout the specification may be used for the nucleic acid amplification test.

In step S410, the obtaining unit 110 may obtain a detection result(s) of a target analyte(s) of a plurality of reaction wells in a plate. As described above, the obtaining unit 110 according to an embodiment may receive the detection result from the detection result providing device 200, or may load the detection result pre-stored in the memory 120.

In an embodiment, the detection result may include at least some of a plate characteristic (e.g., N x M), a plate identifier, a reaction well identifier, and status information (e.g., positive/negative information) for each reaction well. In another embodiment, the detection result may further include position data for each reaction well, and for example, a coordinate value for the position of the reaction well that may be expressed in a two-dimensional orthogonal coordinate system may be included as the position data for each reaction well. Here, the X-axis and the Y-axis of the two-dimensional orthogonal coordinate system represent the position of the reaction well in the X-axis direction and the position of the reaction well in the Y-axis direction in the plate based in the plate characteristics (e.g., N x M), respectively.

In an embodiment, the status information for each reaction well may directly or indirectly include information on whether each reaction well is positive or negative. For example, the status information for each reaction well may include predetermined data (e.g., positive:1, negative:0) directly indicating that each reaction well is positive or negative. As another example, the status information for each reaction well may include indirect data such as a predetermined signal value or a measurement value (e.g., a cycle threshold ($C_T$) value) used to determine as positive or negative (e.g., presence or absence of a target analyte).

In step S420, the contamination assumption unit 310 may obtain a position data of positive reaction wells in the plate from the obtained detection result. Throughout the specification, a positive reaction well refers to a reaction well determined to be positive among a plurality of reaction wells in the plate according to the detection result, and a negative reaction well refers to a reaction well determined to be negative.

In this case, at least some of the positive reaction wells may be true-positive reaction wells determined to be positive by the target analyte contained in the sample at the time of sampling, or may be false-positive reaction wells determined to be positive due to various causes after sampling even though the target analyte was not contained in the sample at the time of sampling.

In an embodiment, the contamination assumption unit 310 may obtain the position data of the positive reaction well in the plate, from the position data for each reaction well and the status information for each reaction well included in the obtained detection result. For example, the contamination assumption unit 310 may detect a coordinate value of the positive reaction well from a coordinate value of each reaction well based on the positive/negative information of each reaction well.

In another embodiment, the contamination assumption unit 310 may obtain the position data of the positive reaction well in the plate by using the plate characteristic, the reaction well identifier, and the positive/negative information for each reaction well included in the obtained detection result. For example, the contamination assumption unit 310 may generate a coordinate value for a two-dimensional position of the reaction well by using the arrangement structure of the reaction wells of the N x M according to the plate characteristics and the arrangement order of the reaction wells according to the reaction well identifier, and may detect the coordinate value of the positive reaction well from the coordinate value of each reaction well based on the positive/negative information for each reaction well. As another example, the contamination assumption unit 310 may determine positive/negative for each reaction well by applying a measurement value such as a $C_T$ for each reaction well to a pre-stored positive/negative analysis model, and may detect a coordinate value of the positive reaction well in the above-described manner according to a result of the positive/negative determination.

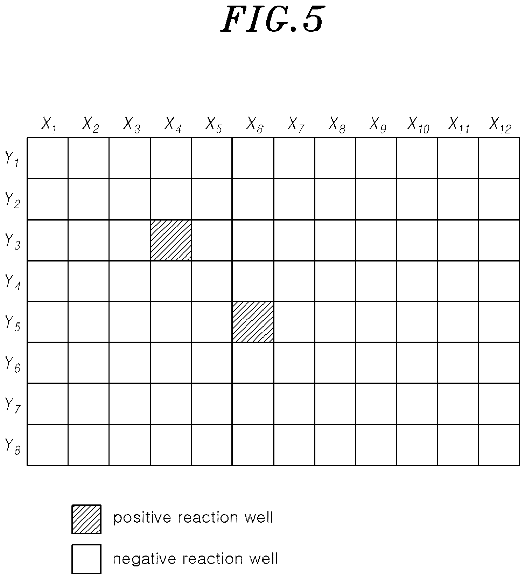

FIG. 5 is a diagram illustrating the position data of the positive reaction wells in the plate according to an embodiment.

Referring to FIG. 5, it is exemplarily shown that 96 reaction wells having 12 x 8 structure are included in the plate and positive reaction wells are located at some points of the plate. FIG. 5 is an example illustrated for convenience of description, and it can be seen that the positive reaction wells in the plate may be distributed in various numbers at various positions according to various detection cases.

In an embodiment, the position data of the positive reaction well in the plate may be expressed through a coordinate value for the position of the positive reaction well or a multi-dimensional chart (e.g., an image, a graph, a matrix), and the like, but is not limited thereto. In an example, for a plate including reaction wells of N x M, when the position of each reaction well is expressed as a two-dimensional coordinate value (X, Y), the coordinate values of the positive reaction well in FIG. 4 may be (4, 3) and (6, 5). As another example, when expressed by three-dimensional coordinate values (X, Y, Z) (here, Z represents a positive/negative result, for example, positive is 1 and negative is 0), and in FIG. 4, the coordinate values of the positive reaction well may be (4, 3, 1) and (6, 5, 1). As another example, when the position of the reaction well is expressed as a two-dimensional image area, a two-dimensional image area for the position of the reaction well may be defined through a unit pixel corresponding to each basic unit (e.g., 1) of N and M, and in FIG. 5, the position of the positive reaction well may correspond to pixel(s) corresponding to N=4 in the X-axis direction and M=3 in the Y-axis direction in the image area. In this case, the corresponding pixel(s) may be distinguished from the negative reaction well, for example, in such a manner that a visual mark indicating positive is included, a predetermined value (e.g., 1) is associated, or the like.

In step S430, the contamination assumption unit 310 may assume a contamination of the plate by at least partially using the obtained position data of the positive reaction wells. In an embodiment, the contamination assumption unit 310 may assume whether the plate is contaminated based on a degree to which the positions of the positive reaction wells are adjacent to each other in the plate. For example, the contamination assumption unit 310 may assume that contamination of the plate has occurred when a count of positive reaction wells adjacently located within a preset distance (e.g., 2) is not less than a preset first number (e.g., 5), and a total count of positive reaction wells in the plate is not less than a preset second number (e.g., 10).

Here, the assuming the contamination may be interpreted as a concept of determining whether contamination actually occurred in the plate, or as a concept of quantitatively calculating a possibility that at least one of the positive reaction wells in the plate was determined to be positive due to contamination, but is not limited thereto.

In addition, the assuming the contamination of the plate may be understood as a concept including one or more of assuming the contamination based on a unit of plate and assuming the contamination based on a unit of reaction well of the plate. For example, contamination may be assumed for each plate, contamination may be assumed for each positive reaction well in a plate, and contamination may be assumed for each positive reaction well and each plate.

In addition, the contamination may include a false-positive occurrence in at least some of the positive reaction wells due to one or more contamination sources. For example, the contamination may include cross-contamination in which false-positivity occurs due to contamination in a reaction well near a true-positive reaction well, due to a situation in which a sample (or a signal-generation composition) splashes around a nearby reaction well during pipetting for a specific reaction well in a dispensing process. Hereinafter, embodiments based on the false-positive contamination is described, but is not limited to the above-described embodiment, and may be widely interpreted as a concept encompassing various contamination situations caused by various contamination sources that may occur in a laboratory space. Meanwhile, throughout the specification, contamination may comprehensively mean contamination generated in the detection process of a target analyte or in the preparing the detection process, and may include, for example, contamination generated in the process of extracting nucleic acids from a specimen, the process of PCR setup for a nucleic acid amplification reaction, or the process of performing a nucleic acid amplification reaction.

In one embodiment, the one or more contamination sources may include at least one selected from the group consisting of: a first contamination source caused by a nucleic acid molecule extracted from a pathogen in a positive reaction well being propagated into the air; a second contamination source caused by the nucleic acid molecule of a pipette in contact with the positive reaction well; a third contamination source caused by a poor sealing of the reaction well, and combinations thereof. For example, the first contamination source may be caused by nucleic acid molecules contained in the sample of the positive reaction well of true-positive splashed through the air into an adjacent reaction well during the process of dispensing the sample, or by nucleic acid molecules floating in the air within the laboratory space being propagated to the reaction well. In addition, the second contamination source may be caused by the contact of a pipette with an adjacent reaction well during pipetting for a specific reaction well or the splashing of nucleic acid molecules into an adjacent reaction well through the relevant pipette. In addition, the third contamination source may be caused by pathogens contained in a sample of a reaction well being transferred to another reaction well through a sealing film during a detection process as some reaction wells are incompletely sealed due to poor sealing of one surface of the plate or poor sealing of open/close parts of the reaction wells. In addition, a case where two or more of the first to third contamination sources are combined may also occur.

In an embodiment, the contamination assumption unit 310 may calculate an assumed value indicating a possibility that contamination has occurred in one or more positive reaction wells in the plate, based on the count of positive reaction wells located adjacent to each other in the plate and a degree to which the positions are adjacent. Here, the assumed value is a value quantitatively indicating the degree to which the positive reaction wells are adjacent to each other in the plate, and may be used as an index indicating the possibility that contamination has occurred in the plate.

In an embodiment, when the calculated assumed value is not less than a predetermined threshold value, the contamination assumption unit 310 may assume that contamination has occurred or may have occurred in one or more positive reaction wells in the plate. Here, the predetermined threshold value refers to a comparison criterion for the magnitude of the assumed value. The threshold may be obtained in various ways, for example, based on statistical information, which will be described in detail later.

The contamination assumption unit 310 may assume the contamination by using the position data of the positive reaction wells in various ways according to various embodiments. In an embodiment, the contamination assumption unit 310 may determine at least one of (a) a distance between the positive reaction wells, (b) a density of the positive reaction wells, and (c) an area or a shape of a figure defined according to a line connecting the positive reaction wells based on the obtained position data, and may assume the contamination of the plate by using at least one of: the distance; the density;

and the area or the shape of the figure. In another embodiment, the contamination assumption unit 310 may determine the count of positive reaction wells located within a preset distance and the degree of adjacency, based on the position data of the positive reaction wells, and may assume the contamination of the plate by using the determined information. In another embodiment, the contamination assumption unit 310 may assume the contamination of the plate by using the position data of the positive reaction wells and a positive-negative determination basis. In another embodiment, the contamination assumption unit 310 may assume a contamination in each of the plurality of the positive reaction wells by using the obtained position data, and may assume the contamination of the plate by using the assumed result of the contamination in each of the positive reaction wells.

Various embodiments for assuming contamination of the plate presented above will be described in more detail below.

1) Assuming contamination of a plate by using a distance between positive reaction wells

Referring to FIG. 6, the contamination assumption unit 310 according to the first embodiment may determine a distance between the positive reaction wells in the plate based on the position data of the positive reaction wells, and may assume the contamination of the plate by using the determined distance.

In an embodiment, the contamination assumption unit 310 may calculate a straight-line distance (e.g., $2\sqrt{2}$) between coordinate values (e.g., $(4, 3)$, $(6, 5)$) of the positive reaction well or a straight-line distance between center points of corresponding pixels, and may calculate an assumed value of contamination based on the calculated straight-line distance and the count of positive reaction wells. For example, the contamination assumption unit 310 may calculate an assumed value for contamination occurrence to be higher as the straight-line distance is shorter and the count of positive reaction wells located within a preset straight-line distance is larger. In addition, when the calculated assumed value is equal to or greater than a predetermined threshold value, the contamination assumption unit 310 may assume that contamination has occurred in the plate.

2) Assuming contamination of a plate by using a density of positive reaction wells

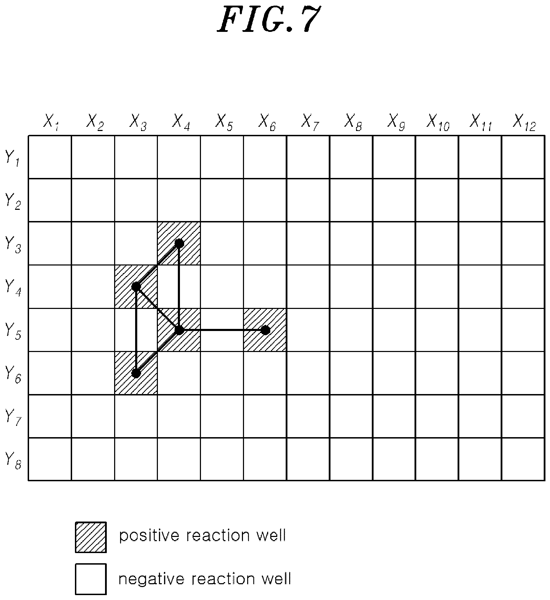

Referring to FIG. 7, the contamination assumption unit 310 according to the second embodiment may determine a density of the positive reaction wells in the plate based on the position data of the positive reaction wells, and may assume the contamination of the plate by using the determined density.

In an embodiment, the contamination assumption unit 310 may generate lines connecting the positions of each positive reaction well to other positive reaction wells within a range of a preset maximum connection distance (e.g., 2), and may calculate an assumed value for contamination based on the count of generated lines and the length of each line. For example, the contamination assumption unit 310 may calculate a density of each positive reaction well to be higher as the count of connectable lines within the range of the maximum connection distance is larger and the sum of the lengths of the corresponding lines is smaller. In addition, the contamination assumption unit 310 may calculate an assumed value for contamination occurrence to be higher as the sum of the densities of the respective positive reaction wells is larger and the positive reaction wells having densities greater than or equal to a preset value are closer to each other. Alternatively, when one or more positive reaction wells having density greater than or equal to a predetermined threshold value are detected, the contamination assumption unit 310 may assume that contamination has occurred in the plate.

The above-described embodiment is an example, and various algorithms for calculating the density may be applied.

3) assuming contamination of a plate by using an area or a shape of a figure defined according to a line connecting the positive reaction wells

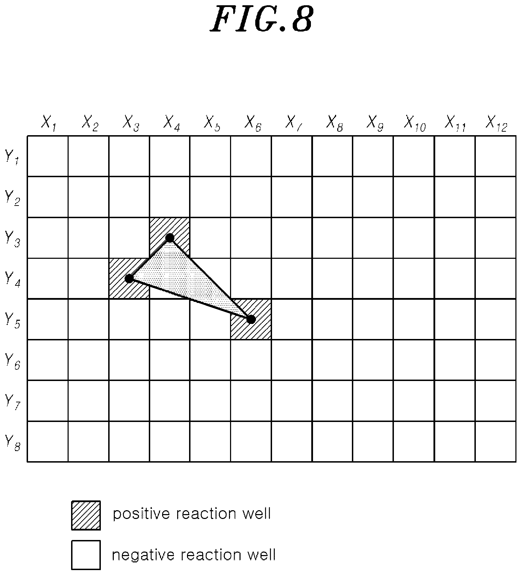

Referring to FIG. 8, the contamination assumption unit 310 according to the third embodiment may determine an area or a shape of a figure defined according to a line connecting the positive reaction wells based on the position data of the positive reaction wells, and may assume the contamination by using the area or the shape of the figure.

In an embodiment, the contamination assumption unit 310 may generate one or more closed polygons along lines connecting the positions of the plurality of positive reaction wells. For example, as shown in FIG. 8, a triangle having different lengths of each side may be generated.

In an embodiment, the contamination assumption unit 310 may calculate an assumed value for contamination based on an area of the generated one or more polygons. For example, the contamination assumption unit 310 may calculate an assumed value for contamination occurrence to be higher as an area of the generated polygon is smaller, the count of generated polygons is larger, and an overlapping area between two or more polygons is larger.

In an embodiment, the contamination assumption unit 310 may calculate an assumed value of contamination based on a shape of the generated one or more polygons. For example, the contamination assumption unit 310 may calculate an assumed value for contamination occurrence to be higher as a shape of the generated polygon is closer to a triangle, the triangle is thinner, and the circumcircle is smaller.

The above-described embodiment is an example, and various algorithms for calculating data distribution density using the area or shape of other figures may be applied. For example, Delaunay triangulation method or the like may be used.

4) Assuming contamination of a plate by using at least a part of the above-described distance, density, and area or shape of the figure

The contamination assumption unit 310 according to the fourth embodiment may assume the contamination of the plate by using two or more of the distance between the positive reaction wells, the density of the positive reaction wells, and the area and shape of the figure described above.

In an embodiment, the contamination assumption unit 310 may primarily calculate assumed values of two or more of the distances between the positive reaction wells, the density of the positive reaction wells, the area of the figure, and the shape in the above-described manner, and may obtain an assumed value secondarily calculated as an assumption result of the contamination occurrence by applying each assumed value to a pre-stored equation. For example, a preset same or different weight may be assigned to each of the two or more assumed values, and the assumed value for the contamination occurrence of the plate may be calculated according to the weighted average result by applying the corresponding weight to each assumed value.

5) Assuming contamination of a plate using a sliding window

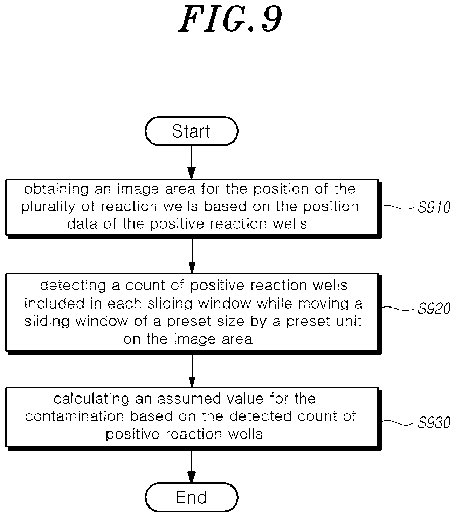

Referring to FIG. 9 to FIG. 11, the contamination assumption unit 310 according to the fifth embodiment may detect a count of positive reaction wells included in each sliding window 20 while moving the sliding window 20 on the image area 10 for the positions of the reaction wells, and may calculate an assumed value for the contamination of the plate based on the detected count.

Specifically, in step S910, the contamination assumption unit 310 according to the fifth embodiment may obtain the image area 10 for the positions of the plurality of reaction wells based on the position data of the positive reaction wells. In an embodiment, the contamination assumption unit 310 may generate the image area 10 of N x M partitioned through a unit pixel corresponding to a basic unit (e.g., 1) of each of N and M, based on the coordinate value for each reaction well or the plate characteristic (e.g., N x M). In addition, the contamination assumption unit 310 may mark the position of the positive reaction well in the image area 10 based on the coordinate value of the positive reaction well or the positive/negative information for each reaction well. For example, as shown in FIG. 9, when the plate including reaction wells of N x M (e.g., 12 x 8) is targeted, the image area 10 may be composed of (N x M) unit pixels corresponding to coordinate values of $(X_N, Y_M)$. In addition, as a visual mark indicating positive is included in the unit pixel corresponding to the position of the positive reaction well(e.g., $(X_4, Y_3)$ and $(X_6, Y_5)$), or as a predetermined value (e.g., 1) is associated with that unit pixel, the position and whether it is positive or negative may be identified or distinguished.

In step S920, the contamination assumption unit 310 according to the fifth embodiment may detect a count of positive reaction wells included in each sliding window 20 while moving the sliding window 20 of a preset size by a preset unit on the image area 10.

Here, the sliding window 20 may be understood as a concept for determining a certain position range while changing a position of the window within the image area 10. In an embodiment, the sliding window 20 may include a first sliding window 21 of a preset first size (e.g., 2 by 2). In another embodiment, the sliding window 20 may further include a second sliding window 22 of a preset second size (e.g., 3 by 3) larger than the first size, but is not limited thereto, and for example, may further include one or more sliding windows of a different size from the above. In addition, the preset size may be set to a value smaller than N or M within the range of N x M, and may be, for example, 2 by 2 or 3 by 3. In addition, the preset unit may be set based on the unit pixel corresponding to the basic unit (e.g., 1) of each of N and M.

The step S920 may be performed by using the first sliding window 21 of the first size (e.g., 2 x 2), and may be performed, for example, according to the following order.

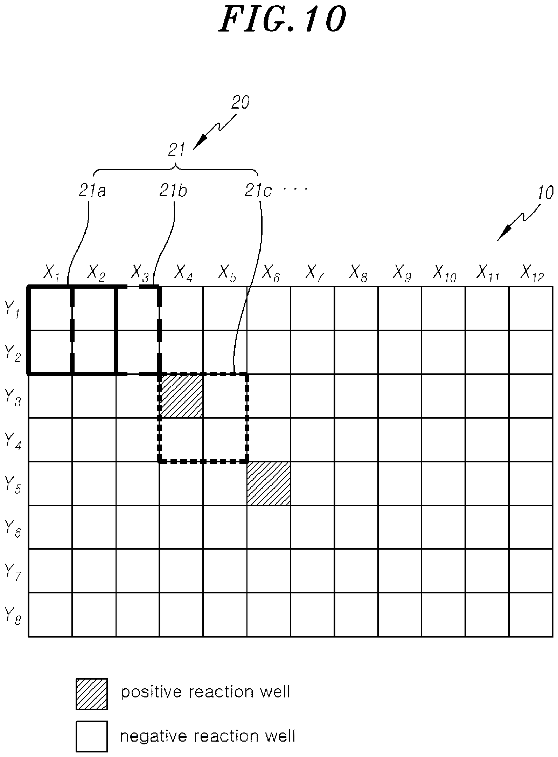

First, the contamination assumption unit 310 may position the first sliding window 21 at a preset start position (e.g., $(X_1, Y_1)$) on the image area 10, and may detect a count of positive reaction wells in the first sliding window 21. For example, in FIG. 10, when the first sliding window 21 is positioned such that the unit pixel of the start position $(X_1, Y_1)$ is positioned at the left upper end (see identifier 21a), the count of positive reaction wells in the first sliding window 21 at the corresponding position is 0.

Next, the contamination assumption unit 310 may move the position of the first sliding window 21 by the preset unit and detect a count of positive reaction wells in the first sliding window 21 at the moved position. For example, when the position of the first sliding window 21 moved by one unit pixel in the X-axis direction (or the Y-axis direction) is $(X_2, Y_1)$ (or $(X_1, Y_2)$) (see identifier 21b), the count of positive reaction wells in the first sliding window 21 at the corresponding position is 0.

In an embodiment, the sliding window 20 may be moved on the image region 10 in the preset unit smaller than the preset size, or may be moved on the image region 10 such that a portion of the positive reaction well included in the sliding window before moving is included in the sliding window after moving. For example, the unit in which the sliding window 20 of n x n size is moved may be 1 when n is 2, and may be 1 or 2 when n is 3. As another example, when the position of the first sliding window 21 before moving of the preset unit is $(X_1, Y_1)$ (see identifier 21a) and the position after moving is $(X_2, Y_1)$ (see identifier 21b), the first sliding window 21 before and after moving may partially overlap. If $(X_1, Y_1)$, $(X_2, Y_1)$, $(X_1, Y_2)$, and $(X_2, Y_2)$ correspond to the positions of the positive reaction well, $(X_2, Y_1)$ and $(X_2, Y_2)$, which are parts of the positive reaction well included in the first sliding window 21 before moving, may be included in the first sliding window 21 after moving. For this purpose, the preset unit may be smaller than the preset size. For example, when the first size of the first sliding window S21 is 2 by 2, the preset unit for moving the first sliding window 21 may be 1, which is smaller than 2.

Thereafter, the above-described process of moving the first sliding window 21 and detecting the count of positive reaction wells may be repeatedly performed. Accordingly, the count of positive reaction wells in the first sliding window 21 may be detected for each of all cases in which the first sliding window 21 of the first size moving in a preset unit can be positioned. For example, with respect to a 12 by 8 reaction well, when it is assumed that the first sliding window 21 of a size of 2 by 2 is moved by one space in a unit pixel, the count of cases in which it can be positioned in the X-axis direction is 11 (=12-1) and the count of cases in which it can be moved in the Y-axis direction is 7 (=8-1). Accordingly, it can be seen that the count of all cases in which the first sliding window 21 can be positioned is 77 (=11x8), and the count of positive reaction wells for each of the 77 cases may be detected (see identifier 21c).

Step S920 may be performed using the second sliding window 22 of the second size (e.g., 3 by 3). As described above, the contamination assumption unit 310 may position the second sliding window 22 at the preset start position (e.g., $(X_1, Y_1)$) on the image area 10, and may detect the count of positive reaction wells in the second sliding window 22 for each of all cases in which the second sliding window 22 moving in a preset unit can be positioned. For example, when it is assumed that the second sliding window 22 of a 3 by 3 size is moved by 1 space in a unit pixel for 12 x 8 reaction wells, it can be seen that the count of all cases in which the second sliding window 22 can be positioned is 60 (= (12-2) x (8-2)), and the count of positive reaction wells may be detected for each of the 60 cases (see identifiers 22a to 22c).

The step S920 may be performed by using the sliding window 20 of the preset size and then performed by using each of the at least one sliding windows 20 of a size different from the preset size. For example, as described above, after the count of positive reaction wells in the first sliding window 21 is detected for 77 cases by using the first sliding window 21 of the first size (e.g., 2 by 2), the count of positive reaction wells in the second sliding window 22 may be detected for 60 cases by using the second sliding window 22 of the second size (e.g., 2 by 2).

In step S930, the contamination assumption unit 310 according to the fifth embodiment may calculate an assumed value for contamination of the plate based on the detected count of positive reaction wells. In an embodiment, the contamination assumption unit 310 may calculate an assumed value by using the sum of the detected count of positive reaction wells. For example, the contamination assumption unit 310 may calculate the assumed value to be higher as a first value obtained by summing the count of positive reaction wells detected for each of a total of 77 cases according to the first sliding window 21 is higher.

In an embodiment, the assumed value for the contamination of the plate may be calculated based on the count of positive reaction wells included in each sliding window 20 detected by using the sliding window 20 of the preset size and the sliding window 20 of the size different from the preset size. For example, a second value is obtained by summing up the count of positive reaction wells detected for each of a total of 60 cases according to the second sliding window 22, and the assumed value may be calculated higher as the result of summing the first value and the second value is higher.

In an embodiment, the assumed value for the contamination of the plate may be calculated based on a weight assigned to each of the first value and the second value. For example, a weight assigned to the first value may be higher than a weight assigned to the second value, and the assumed value may be calculated according to a weighted average result based on these weights. In this case, by giving a higher weight to a case with smaller window size and closer position, the assumed value for contamination may be more effectively quantified.

In an embodiment, the contamination assumption unit 310 may detect a frequency for each count of positive reaction wells in the sliding window 20. Here, the frequency may refer to a number of times that the same count of positive reaction wells are detected in the sliding window 20 for each count, when the process of moving the sliding window 20 and the detecting the count of positive reaction wells are performed. For example, in the case using the first sliding window 21 of 2 by 2, the count of positive reaction wells in the sliding window 20 detected for each of the 77 cases may be any one of 0 to 4, and the frequency of each count may be detected by counting the count of times each of 0 to 4 is detected.

The following Table 1 shows an example where, out of the total of 77 cases, the frequency of detecting the count of positive reaction wells in the first sliding window 21 as 0 is 69 times, the frequency of detecting the same as 1 is 8 times, the frequency of detecting the same as any one of 2 to 4 is 0 times, according to the above-described embodiment of the first sliding window 21.

[Table 1]

| size of the sliding window | ratio of the count of positive reaction wells in the sliding window to the size | frequency |
|---|---|---|
| 2×2 | 0/4 | 69 |
| 2×2 | 1/4 | 8 |
| 2×2 | 2/4 | 0 |
| 2×2 | 3/4 | 0 |
| 2×2 | 4/4 | 0 |

Similarly, the following Table 2 shows an example where, out of the total of 60 cases, the frequency of detecting the count of positive reaction wells in the second sliding window 22 as 0 is 43 times, the frequency of detecting the same as 1 is 16 times, the frequency of detecting the same as 2 is 1 times, the frequency of detecting the same as any one of 3 to 9 is 0 times, according to the above-described embodiment of the second sliding window 22.

[Table 2]

| size of the sliding window | ratio of the count of positive reaction wells in the sliding window to the size | frequency |
|---|---|---|
| 3×3 | 0/9 | 43 |
| 3×3 | 1/9 | 16 |
| 3×3 | 2/9 | 1 |
| 3×3 | 3/9 | 0 |
| 3×3 | 4/9 | 0 |
| 3×3 | 5/9 | 0 |
| 3×3 | 6/9 | 0 |
| 3×3 | 7/9 | 0 |
| 3×3 | 8/9 | 0 |
| 3×3 | 9/9 | 0 |

In an embodiment, the contamination assumption unit 310 may calculate a degree to which the positive reaction wells are adjacent to each other in the plate, as the assumed value, by using the detected each count of the positive reaction wells in the sliding window 20 as described above, the preset size, and the frequency for each count. For example, the contamination assumption unit 310 may calculate the assumed value to be proportional to the detected each count of the positive reaction wells and the frequency and to be inversely proportional to the preset size.

In an embodiment, when one sliding window 20 is used, the contamination assumption unit 310 may calculate the assumed value for contamination of the plate by using Equation 1 below. For example, referring to Table 1, when the first sliding window 21 of 2 by 2 size is used, the assumed value for contamination may be calculated as 2 (= (0/4)*69 + (1/4)*8 + (2/4)*0 + (0/4)*0 +(0/4)*0). Alternatively, referring to Table 2, when the second sliding window 22 of 3 by 3 size is used, the assumed value for contamination may be calculated as 2 (= (0/9)*43 + (1/9)*16 + (2/9)*1 + (3/9)*0 +(4/9)*0 +(5/9)*0 +(6/9) *0 +(7/9)*0 +(8/9)*0 +(9/9)*0).

[Equation 1]

$$a = \sum_{i=1}^{s} \frac{i}{s} c_i$$

(Here, a represents the assumed value for contamination of the plate; i represents the count of positive reaction wells in the sliding window 20; s represents the size (e.g., N x M) of the sliding window 20; $c_i$ represents the frequency

described above, and represents the frequency where, out of the total of cases according to the sliding window 20, the count of positive reaction wells in the sliding window 20 is detected as the i)

In another embodiment, when two sliding windows 20 having different sizes are used, the contamination assumption unit 310 may calculate the assumed value for contamination of the plate by using Equation 2 below. According to an embodiment, when three or more sliding windows 20 are used, Equation 2 may be extended and applied based on the above-described technical idea. Referring to the above example, when the first sliding window 21 and the second sliding window 22 are used, the assumed value for contamination of the plate may be calculated as 4 which is obtained by summing an assumed value (= 2) using the first sliding window 21 and an assumed value (= 16/9 + 2/9) using the second sliding window 22.

[Equation 2]

$$a = \sum_{i=1}^{s_1} \frac{i}{s_1} c_i + \sum_{j=1}^{s_2} \frac{j}{s_2} c_j$$

(Here, a represents the assumed value for contamination of the plate; i and j represent the count of positive reaction wells in the first sliding window 21 and the count of positive reaction wells in the second sliding window 22, respectively; $s_1$ and $s_2$ represent the size of the first sliding window 21 (e.g., 4 (=2 x 2)) and the size of the second sliding window 22 (e.g., 9 (=3 x 3)), respectively; $c_i$ and $c_j$ are the above-described frequencies, and represent the frequency where the count of positive reaction wells in the first sliding window 21 is detected as the i from among the total of cases according to the first sliding window 21, and the frequency where the count of positive reaction wells in the second sliding window 22 is detected as the j from among the total of cases according to the second sliding window 22, respectively;) As described above, by using the sliding window 20, the count of positive reaction wells located within a predetermined distance in the plate may be efficiently quantified. In addition, by using the frequency for each count of positive reaction wells, the count of adjacent positive reaction wells may be effectively quantified. In addition, by using the size of the sliding window 20 as a kind of weight, the degree to which the positive reaction wells are adjacent to each other may be effectively quantified.

6) Assuming contamination of a plate by using an interquartile range (IQR)-based threshold value

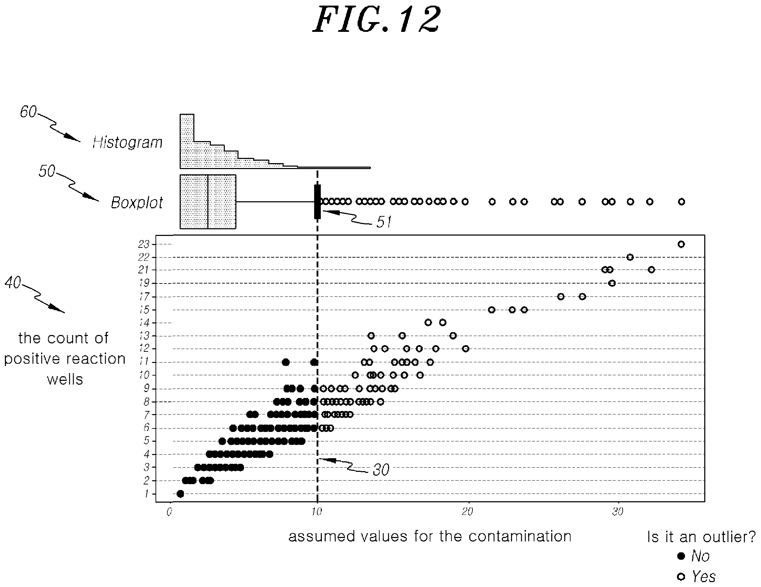

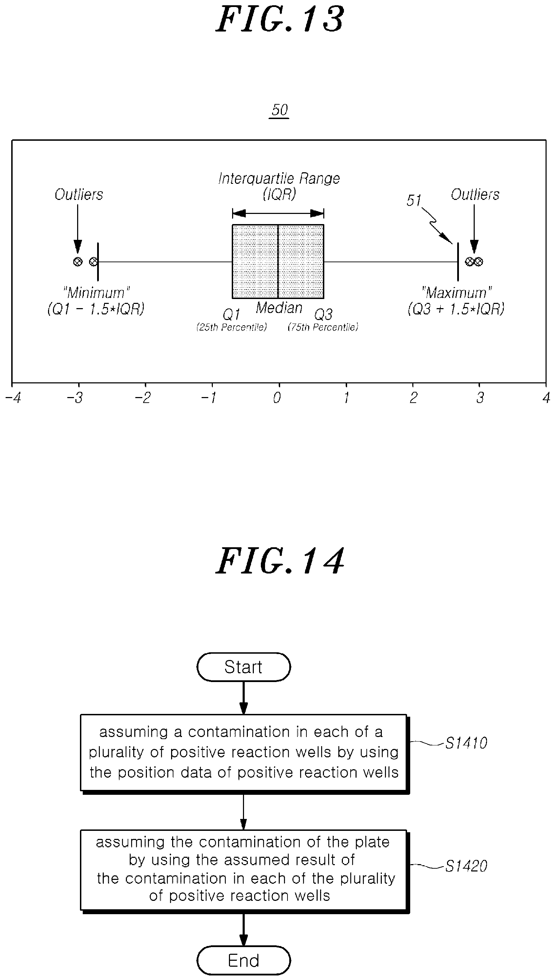

While the first to fifth embodiments described above are embodiments related to a method for calculating the assumed value for contamination of the plate, the sixth embodiment is an embodiment related to a method for setting a threshold value which is a comparison criterion of the assumed value or assuming contamination of the plate by using the threshold value. Referring to FIG. 12 and FIG. 13, the contamination assumption unit 310 according to the sixth embodiment may obtain an IQR-based threshold value 30, and assume the contamination of the plate based on a result of comparing the assumed value of contamination with the obtained threshold value 30.

In FIG. 12, a first graph 40 is a graph illustrating a change in the count of positive reaction wells according to a change in the assumed value for contamination of the plate. Here, the count of positive reaction wells means the total count of positive reaction wells in the corresponding plate.

A plurality of points shown in the first graph 40 exemplarily represent that a plurality of assumed values for the contamination calculated for a plurality of plates expressed as coordinate values in the X-axis direction, and the count of positive reaction wells detected in each plate expressed as coordinate values in the Y-axis direction. For example, a plurality of detection results for a plurality of plates may be obtained in one or more laboratory spaces that are the same or different, and a plurality of assumed values may be calculated by using the plurality of detection results. A dataset for the assumed value including the plurality of assumed values may be stored and managed, may be periodically updated to include additionally calculated assumed values.

In the first graph 40, the threshold value 30 is exemplarily illustrated. In an embodiment, when the assumed value (e.g., 12) calculated for the specific plate is greater than the threshold value 30 (e.g., 10), the contamination assumption unit 310 may assume that contamination has occurred in the corresponding plate.

The second graph 50 is a box plot-based statistical graph illustrating a statistical distribution for the plurality of assumed values described above. In an embodiment, the contamination assumption unit 310 may determine the threshold value 30 based on the IQR and a third quartile Q3 shown in the second graph 50. The second graph 50 will be described in more detail together with the box plot algorithm illustrated in FIG. 13.

In FIG. 13, the second graph 50 may include a first quartile Q1, a median, and a third quartile, which are obtained from the dataset for the assumed value. For example, the first quartile, the median, and the third quartile indicate values corresponding to 25%, 50%, and 75%, respectively, when the plurality of assumed values included in the dataset are sorted in ascending order in size order. In addition, the second graph 50 may include a box whose ends are the first quartile and the third quartile, and the median of the box may be indicated by a vertical line. In this case, IQR refers to a horizontal length of the box, and may be calculated, for example, by subtracting the first quartile from the third quartile.

In addition, a minimum value and a maximum value 51 may be values corresponding to left and right ($\pm 1.5$*IQR) from the first quartile and the third quartile, respectively. For example, when the assumed value of the contamination exceeds the maximum value 51 or is less than the minimum value, it may be determined to correspond to an outlier point.

The dataset for the assumed value obtained according to an embodiment shows an exemplary measurement result in which the first quartile is statistically very small and the box is concentrated on the left side, as shown in a second graph 50 of FIG. 12. Considering this measurement result, when the threshold value 30 is determined by using the maximum value 51, the contamination may be assumed more effectively.

In an embodiment, the contamination assumption unit 310 may determine the threshold value 30 based on the IQR, the third quartile, and a predetermined weight. For example, the contamination assumption unit 310 may determine the threshold value 30 based on Equation 3 below.

$$[\text{Equation 3}]$$

$$T = Q_3 + w * IQR$$

(Here, 'T' represents the threshold value 30, '$Q_3$' represents the third quartile, 'w' represents the predetermined weight, and 'IQR' represents the IQR obtained from the dataset for the assumed values of the plurality of plates described above)

In an embodiment, the predetermined weight may be set or updated by an user. For example, when an assumed value is calculated according to a general case, the weight may be set to an initial value of 1.5 and may be equal to the maximum value 51. If a stricter criterion is to be applied to the assumed value, the weight may be increased by a preset ratio relative to the initial value, which will result in a relatively larger count of plates being assumed to be contaminated as the threshold value 30 increases. If a looser criterion is to be applied to the assumed value, the weight may be decreased by the preset ratio relative to the initial value, which will result in a relatively smaller count of plates being assumed to be contaminated as the threshold value 30 is decreased.

In order to enable easy check of statistical distributions, the second graph 50 may be scaled according to the assumed value for contamination appearing on the X-axis of the first graph 40 and displayed together on one plane as shown in FIG. 12. As shown in FIG. 12, since most of the measured values are on the left side of the X-axis, the box in the second graph 50 concentrated on the left, and accordingly, the difference between the minimum value and the first quartile may be very small. In addition, the threshold value 30 may be set to match the maximum value 51 indicated in the second graph 50, and may be adjusted upward or downward according to an embodiment.

A third graph 60 is a statistical graph showing a distribution of the assumed values as histogram. Similarly, for easy check of the statistical distribution, the third graph 60 may be scaled according to the assumed value for contamination appearing on the X-axis of the first graph 40 and displayed together on one plane as shown in FIG. 12.

In an embodiment, the contamination assumption unit 310 may directly determine the threshold value 30 according to the above-described embodiment. In another embodiment, the operation of determining the threshold value 30 may be performed by a device other than the computer device 100 (e.g., a management server that stores and manages detection results collected from a plurality of laboratory spaces, a user terminal, etc.), and the computer device 100 may receive the threshold value 30 from the other device and use it for assuming contamination of a plate. In addition, the threshold value 30 may be set based on various statistical algorithms in addition to the box plot algorithm.

Meanwhile, in a specific season (e.g., winter) in which a certain disease is mainly prevalent, or in a situation in which a specific disease (e.g., COVID-19) is prevalent, the assumption accuracy of contamination of the plate may be relatively reduced. This is because there may be differences in the detection frequency of specific pathogens according to various situations that may be at the time of testing, and accordingly, there may be differences in the statistical distribution for the count of positive reaction wells in the plate or the assumed values. The present disclosure may have a greater effect when it is implemented to assume contamination of a plate tested under a general situation without issues such as a season or a disease prevalence, but is not limited thereto.

As described above, since the assumed value of contamination may vary depending on the positive rate at a specific period and region, it is recommended to check the assumed values within the same institution and within the same period for more accurate assumption of contamination and/or quality control of the laboratory space to be described below. However, the present disclosure is not limited thereto. For example, in the case of COVID-19, since the recent positive rate in 2022 is generally much higher than the positive rate in 2021, when comparing assumed values by setting the period affecting the overall positive rate as a control variable, the assumption accuracy may be improved more than when comparing assumed values by setting the two periods as variables at the same level.

The threshold value 30 according to an embodiment may be classified into a predetermined category. Here, the predetermined category is to apply an optimal threshold value 30 according to the above-described various situations. In an embodiment, a dataset including a plurality of assumed values calculated from a plurality of detection results may be classified into a plurality of categories including a season prevalence, a disease prevalence, a type of a laboratory space, and an identifier of the laboratory space. For the classified assumed values, a threshold for each category may be calculated as the same or different value according to the above-described embodiment. In addition, in the process for assuming contamination of a specific plate, when the test time point at which the detection process for the plate is performed is not a general situation, a threshold value for each category corresponding to the specific situation at the corresponding test time point among the plurality of threshold values for each category may be loaded and used as the threshold value 30.

In an embodiment, the contamination assumption unit 310 may assume contamination of the plate by using the count of positive reaction wells, the assumed value of contamination, and the threshold value 30. In this regard, referring to the first graph 40, it can be seen that the assumed value of the contamination of the plate and the count of positive reaction wells are relatively proportional to each other. For example, as the count of positive reaction wells increases, it can be seen that the assumed values calculated from various plates may vary and the assumed values of contamination move upward to the right in a direction in which the value range relatively increases according to the count of positive reaction wells. Accordingly, when assuming the contamination of the plate, if the comparison result between the count of positive reaction wells and the assumed value of contamination are considered together with the comparison result between the above-described assumed value and the threshold value 30, the reliability of the assumption result may be further improved.

In an embodiment, the contamination assumption unit 310 may assume that contamination has occurred in the plate when both the comparison condition between the assumed value and the threshold value 30 and the ratio condition between the count of positive reaction wells and the assumed value of contamination are satisfied. For example, when the ratio (e.g., 7/11) of the count (e.g., 7) of positive reaction wells to the assumed value is less than a preset value (e.g., 1), the contamination assumption unit 310 may assume that no contamination has occurred, even though the assumed value (e.g., 11) is greater than the threshold value 30 (e.g., 10). In another embodiment, the contamination assumption unit 310 may assume that contamination has occurred even when only one of the comparison condition and the ratio condition is satisfied.

In an embodiment, the contamination assumption unit 310 may obtain a re-detection result of the target analyte of the positive reaction well in the plate, and update an equation used to assume the contamination and/or the threshold value 30 which is a criterion for assuming the contamination, based on a comparison result between the re-detection result and the detection result. For example, with respect to a specific plate assumed to be contaminated, a plurality of samples used to a plurality of reaction wells (or positive reaction wells) in the corresponding plate may be used again, or new samples re-sampled from the examinees of the corresponding sample may be used to perform the detection process again, and thus the re-detection result may be obtained. The contamination assumption unit 310 may compare the positive/negative information for each reaction well, the count or position of positive reaction wells, the assumed value of contamination, etc., which are obtained from each of the re-detection result and the detection result respectively, and then determine whether there is a difference between them. When there is the difference, the contamination assumption unit 310 may update one or more of the equations described throughout the specification based on the directionality represented by the difference. For example, when contamination occurrence is assumed and re-tested, but the difference described above is 0 or larger than a preset level, parameters in the equations may be partially modified.

In an embodiment, the contamination assumption unit 310 may determine that the reaction well determined to be positive in the detection result but negative in the re-detection result is a contaminated reaction well in which contamination has occurred. For example, the contaminated reaction well may correspond to a reaction well in which a false-positive occurs due to a contamination source such as pipetting in the dispensing process of the sample, and whether the reaction well is a contaminated reaction well may be determined by comparing a positive/negative determination result between the detection result and the re-detection result.

In an embodiment, when any one positive reaction well among the positive reaction wells is determined to be positive in the detection result but is determined to be negative in the re-detection result, the contamination assumption unit 310 may update the equation used to assume the contamination and/or the threshold value 30 based on the difference in assumed values of the contamination calculated from each of the re-detection result and the detection result. In an embodiment, the contamination assumption unit 310 may update the equation used to assume the contamination and/or the threshold value 30, based on the difference in the assumed values calculated from the detection results and the re-detection result, respectively, and whether the contaminated reaction well is detected. For example, when the contaminated reaction well is detected from the re-detection result but two assumed values calculated from the re-detection result and the detection result, respectively, are greater than (or smaller than) the threshold value 30, the contamination assumption unit 310 may update Equation 1 or 2 in a direction of decreasing (or increasing) the assumed value, or may update the value of the weight w used in Equation 3 in a direction of increasing (or decreasing) the threshold value 30. As another example, when the contaminated reaction well is not detected from the re-detection result but any one of the two assumed values calculated from the re-detection result and the detection result, respectively, is greater than the threshold value 30, the contamination assumption unit 310 may update the equation in a direction of decreasing the assumed value or increasing the threshold value 30.

7) Assuming contamination in unit of positive reaction well, and assuming contamination in unit of plate based on the assumption result in unit of positive reaction well

While the first to fifth embodiments described above are embodiments related to the method for calculating the assumed value for contamination in unit of plate, the sixth embodiment is an embodiment related to a method calculating a first assumed value for contamination possibility in unit of positive reaction well and then calculating a second threshold value for contamination possibility in unit of plate based on the first assumed value in unit of the positive reaction well.

Referring to FIG. 14, in step S1410, the contamination assumption unit 310 according to the seventh embodiment may assume a contamination in each of the positive reaction wells by using the position data of the positive reaction wells. In an embodiment, the contamination assumption unit 310 may calculate a plurality of the first assumed values by quantifying a degree to which each of the plurality of positive reaction wells are adjacent to each other, and may assume whether each of the plurality of positive reaction wells is contaminated based on the plurality of the first assumed values.

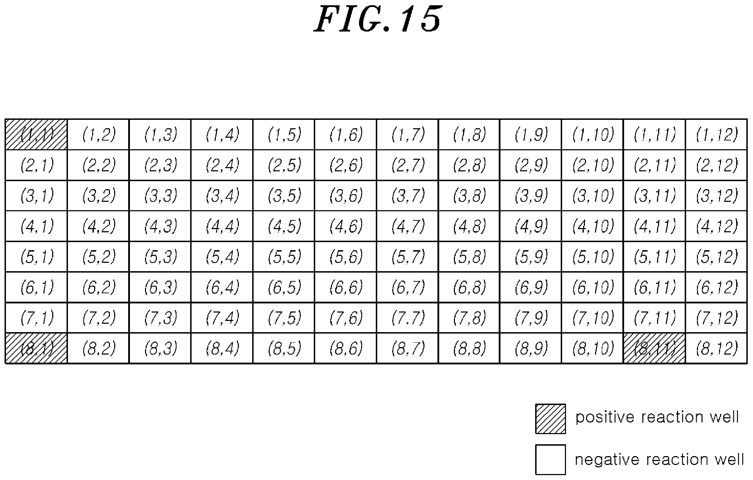

As described above, the position data of the positive reaction well may include the coordinate value (X, Y) for the position of each reaction well and the positive/negative result for each reaction well. FIG. 15 to FIG. 17 show the coordinate value of each reaction well on the plate of 12 x 8 structure, and show examples of a case in which three positive reaction wells are most dispersed, a case in which the positive reaction wells are positioned partially adjacent to each other, and a case in which the positive reaction wells are positioned most adjacent to each other. In an embodiment, a certain coordinate value (e.g., (7,12), (8,12)) may not be used in the process of calculating the assumed value, as it corresponds to a negative control (NC), a positive control (PC), or the like.

Referring to FIGS. 15 to 17, the contamination assumption unit 310 may determine the distance between the positive reaction wells based on the position data of the positive reaction well. Here, the distance between the positive reaction wells indicates the distance between each positive reaction well and the other positive reaction wells. For example, the contamination assumption unit 310 may calculate a distance between a first positive reaction well and each of the other positive reaction wells to assume a contamination of the first positive reaction well, and may calculate a distance between a second positive reaction well and each of the other positive reaction wells to assume a contamination of the second positive reaction well.

In an embodiment, the distance between the positive reaction wells may be calculated based on a Manhattan distance measurement method. Specifically, the distance between the positive reaction well at the coordinate value $(x_1, y_1)$ and the positive reaction well at the coordinate value $(x_2, y_2)$ may be calculated as $|x_1 - x_2| + |y_1 - y_2|$, for example, the Manhattan distance between the positive reaction well at the coordinate value (1,1) and the positive reaction well at the coordinate value (8,1) is 7. Accordingly, various Manhattan distances may be measured within a range from a minimum distance 1 (e.g., a distance between (1,1) and (1,2)) to a maximum distance 18 (e.g., a distance between (1,1) and (8,12)). In another embodiment, the distance between the plurality of positive reaction wells may be calculated based on the Euclidean distance measurement method, and for example, may be calculated as the length of a straight line connecting each coordinate value as described above with respect to the first embodiment.

In an embodiment, the contamination assumption unit 310 may calculate each of a plurality of the first assumed values indicating a contamination possibility in each of the positive reaction wells based on the determined distance. In an embodiment, each of the plurality of the first assumed values may be determined by at least partially using a result of summing reciprocals of the distance between each positive reaction well and other positive reaction wells. For example, when there are two or more positive reaction wells in the plate, each distance from each positive reaction well to another positive reaction well may be calculated, and the first assumed value may be calculated by using a reciprocal of each distance.

In an embodiment, each of the plurality of the first assumed values may be calculated based on Equation 4 below. In Equation 4, $a_1(x, y)$ denotes the first assumed value $a_1$ of the positive reaction well at the coordinate value (x, y), $d_m$ denotes a maximum distance (e.g., a maximum Manhattan distance is 18 in the case of the plate having a 12 x 8 structure) that can be calculated from the coordinate value (x, y), $d_i$ denotes the Manhattan distance (e.g., $d_i=i$) between the positive reaction wells according to i, and $c_i$ denotes the count of all positive reaction wells spaced apart from each other by a distance of di in the plate.

[Equation 4]

$$a_{1(x,y)} = \sum_{i=1}^{d_m} \frac{1}{d_i} c_i$$

As a specific example, as shown in FIG. 15, when three positive reaction wells are most dispersed, the first assumed value of the positive reaction well at the coordinate value (1, 1) may be about 0.202, which is the sum of the reciprocal (=1/7) of the distance from the coordinate value (8, 1) and the reciprocal (=1/17) of the distance from the coordinate value (8, 11). In addition, the first assumed value of the positive reaction well at the coordinate value (8, 1) may be about 0.243, which is the sum of the reciprocal (=1/7) of the distance from the coordinate value (1, 1) and the reciprocal (=1/10) of the distance from the coordinate value (8, 11). In addition, the first assumed value of the positive reaction well at the coordinate value (8, 11) may be about 0.159, which is the sum of the reciprocal (=1/17) of the distance from the coordinate value (1, 1) and the reciprocal (=1/10) of the distance from the coordinate value (8, 1).

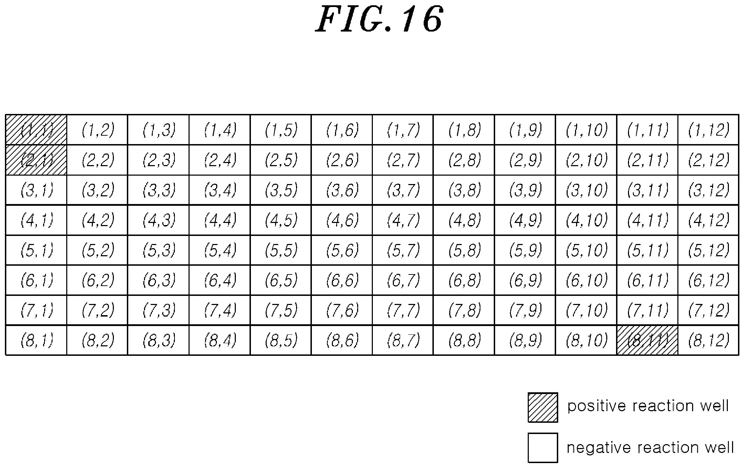

In addition, as shown in FIG. 16, when the three positive reaction wells are positioned partially adjacent to each other, the first assumed value of the positive reaction well at the coordinate value (1, 1) may be about 0.559, which is the sum of the reciprocal (=1/2) of the distance from the coordinate value (3, 1) and the reciprocal (=1/17) of the distance from the coordinate value (8, 11). In addition, the first assumed value of the positive reaction well at the coordinate value (3, 1) may be about 0.567, which is the sum of the reciprocal (=1/2) of the distance from the coordinate value (1, 1) and the reciprocal (=1/15) of the distance from the coordinate value (8, 11). In addition, the first assumed value of the positive reaction well at the coordinate value (8, 11) may be about 0.126, which is the sum of the reciprocal (=1/15) of the distance from the coordinate value (3, 1) and the reciprocal (=1/17) of the distance from the coordinate value (1, 1).

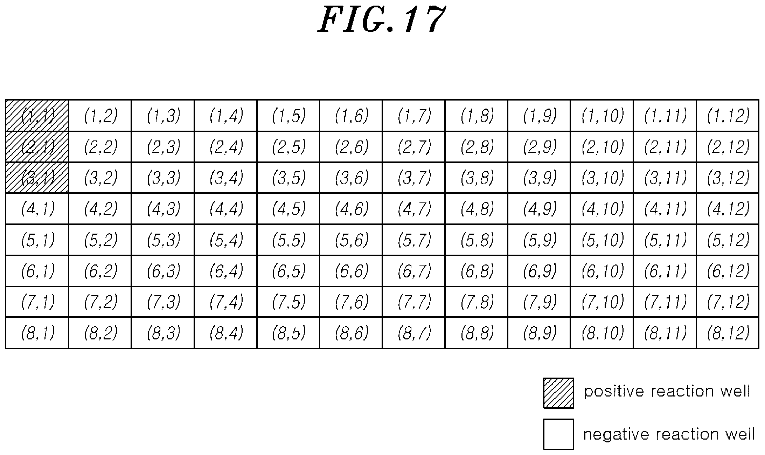

In addition, as shown in FIG. 17, when the three positive reaction wells are positioned closest to each other, the first assumed value of the positive reaction well at the coordinate value (1, 1) may be 1.5, which is the sum of the reciprocal (=1/1) of the distance from the coordinate value (2, 1) and the reciprocal (=1/2) of the distance from the coordinate value (3, 1). In addition, the first assumed value of the positive reaction well at the coordinate value (2, 1) may be 2, which is the sum of the reciprocal (=1/1) of the distance from the coordinate value (1, 1) and the reciprocal (=1/1) of the distance from the coordinate value (3, 1). In addition, the first assumed value of the positive reaction well at the coordinate value (3, 1) may be 1.5, which is the sum of the reciprocal (=1/2) of the distance from the coordinate value (1, 1) and the reciprocal (=1/1) of the distance from the coordinate value (2, 1).

In general, since the contamination possibility decreases as the distance between the positive reaction wells increases, when the reciprocal of the distance is used as described above, the assumed value may be calculated to be proportional to the contamination possibility.

In an embodiment, the contamination assumption unit 310 may assume the contamination of each of the plurality of positive reaction wells based on each of the plurality of the first assumed values. For example, when the first assumed value of a certain positive reaction well is not less than a preset first threshold value, the contamination assumption unit 310 may assume that the false-positive has occurred in the corresponding positive reaction well.

As a specific example, in the case of FIG. 15 and FIG. 16 described above, since all of the first assumed values for each positive reaction well are less than the first threshold value (e.g., 2), it may be assumed that no contamination has occurred. However, in the case of FIG. 17, the positive reaction well at the coordinate value (2, 1) not less than the first threshold value (e.g., 2) may be assumed that contamination has occurred.

In an embodiment, the first threshold value, which is a comparison criterion of the first assumed value, may be included in the threshold value 30, and may be set through, for example, box plot-based statistical analysis on the dataset of the first assumed value according to the above-described embodiment. In addition, the first threshold value may be classified according to the position characteristics (e.g., corner, center, near PC, etc.) of the positive reaction well. For example, when the first assumed value is compared with the first threshold value, the same or different first threshold value may be applied according to a predetermined category corresponding to the position of the corresponding positive reaction well.

In step S1420, the contamination assumption unit 310 may assume the contamination of the plate based on the result of assuming the contamination of each of the plurality of positive reaction wells. In an embodiment, the contamination assumption unit 310 may calculate a second assumed value indicating a contamination possibility of the plate by using the result of summing the plurality of the first assumed values. For example, when the second assumed value is not less than a preset second threshold value, the contamination assumption unit 310 may assume that the false-positive has occurred in one or more positive reaction wells in the plate.

As a specific example, the second assumed value is 0.603 (=202+0.243+0.159) in the case of FIG. 15, the second assumed value is 1.251 (=0.559+0.567+0.126) in the case of FIG. 16, and the second assumed value is 5 (=1.5+2+1.5) in the case of FIG. 17. In the case of FIG. 15 and FIG. 16, since the second assumed value is smaller than the second threshold value (e.g., 5), it may be assumed that no contamination has occurred in the plate. However, in the case of FIG. 17, since the second assumed value is greater than or equal to the second threshold value (e.g., 5), it may be assumed that contamination has occurred in the plate.

In an embodiment, the second assumed value may be calculated by using a weighted average based on the same or different weights assigned to each of the plurality of the first assumed values according to the position of each positive reaction well. For example, when the positive reaction well is positioned near the PC or NC where contamination occurs relatively more frequently, or when is positioned in a corner where the count of adjacent reaction well is relatively small, a relatively large weight may be given.

In an embodiment, the second threshold value, which is a comparison criterion of the second assumed value, may be included in the threshold value 30. For example, the second threshold may be set through box plot-based statistical analysis on the dataset of the second assumed value according to the above-described embodiment.

8) Assuming contamination in unit of positive reaction well

The contamination assumption unit 310 according to the eighth embodiment may assume the contamination of the plate by assuming the contamination of each of the plurality of positive reaction wells. In the eighth embodiment, "the contamination of the plate (or whether the plate is contaminated)" may refer to " the contamination of the each of the plurality of positive reaction wells (or whether each of the plurality of positive reaction wells is contaminated)". For example, the operation of assuming the contamination possibility for each positive reaction well by calculating the first assumed value for each positive reaction well is performed, but the operation of assuming the contamination for each plate by calculating the second assumed value for the entire plate may be omitted.

9) Assuming contamination of a plate by using a positive-negative determination basis

While the first to eighth embodiments described above are embodiments related to a method for assuming contamination by using the position data of the positive reaction wells, the ninth embodiment is an embodiment related to a method for assuming the contamination by additionally using the positive-negative determination basis as well as the position data of the positive reaction wells.

Before describing this, a case will be exemplarily described where a portion of a sample containing a high concentration of target analytes in a true-positive reaction well splashes into one or more adjacent true-negative reaction wells due to the above-described contamination source, resulting in contamination. In this case, the corresponding portion of sample included in the true-positive reaction well may be diluted into the true-negative reaction well. This may result in false-positive, where the reaction well that was originally negative is falsely detected as having the target analyte during the nucleic acid amplification reaction. In this case, when a cycle threshold (Ct) value, which is a basis for determining positive or negative of the reaction well, is measured in the amplification graph obtained from the detection result and compared them, it can be inferred that the Ct value of the true-positive reaction well will be still low due to the high concentration of the positive sample, but the Ct value of the false-positive reaction well that was negative originally will be large due to the relatively low concentration. Based on this inference result, when the contamination is assumed by additionally using the Ct value between the positive reaction well and the adjacent positive reaction well, it is possible to implement higher assumption accuracy than when only the position data of the positive reaction wells is used.

Hereinafter, according to the ninth embodiment, various embodiments of assuming the contamination of the plate by using this positive-negative determination basis of the reaction wells together with the position data of the positive reaction wells will be described. The following embodiments may be understood with reference to the first to eighth embodiments described above.

In an embodiment, the contamination assumption unit 310 may obtain the positive-negative determination basis of the reaction wells from the detection result. Here, the positive-negative determination basis represents a basis that can be used in the process of determining positive indicating the presence of the target analyte in the reaction well or negative indicating the absence of the target analyte in the reaction well, and may be, for example, one or more signal values, measured values, or calculated values used as a determination criterion for positive or negative.

In an embodiment, the positive-negative determination basis may include at least one of (a) a cycle value indicating a time or reaction number for an intensity of a signal value to reach a predetermined threshold value in a set of signal values, and (b) a signal value at a specific cycle in the set of the signal values. According to an embodiment, the positive-negative determination basis may include a derivative result for an amplification curve obtained by connecting points corresponding to the set of the signal values.

In an embodiment, the cycle value may include cycle threshold (Ct), which means a cycle value in which the relative fluorescence unit (RFU) of the amplification reaction for the target analyte reaches a threshold value. In addition, the signal value in the specific cycle may include a magnitude value (e.g., RFU) of a fluorescence signal obtained from a nucleic acid amplification reaction when the cycle value reaches a predetermined cycle value in the amplification graph, a value obtained by processing the magnitude value in a predetermined manner, or the like. In addition, the derivative result may include a cycle value in which the value of the first derivative or the second derivative of the amplification curve satisfies a predetermined criterion (e.g., a maximum value). Hereinafter, for convenience of description, Ct is mainly used as an example of the positive-negative determination basis, but the positive-negative determination basis of the present disclosure is not limited thereto, and may include various other criteria that may be used to determine whether a reaction well is positive or negative.

The above-described positive-negative determination basis may be obtained from the detection result as described above. In an embodiment, the positive-negative determination basis may be already included in the detection result, and for example, positive/negative information for each reaction well and the positive-negative determination basis may be included together as the status information for each reaction well in the detection result. In another embodiment, the positive-negative determination basis may be obtained through a predetermined signal processing on the set of the signal values included in the detection result. For example, the detection result may include the set of the signal values including a signal value for each cycle as a result of the amplification reaction, and the positive-negative determination basis may be obtained by performing a process of deriving the positive-negative determination basis and the positive/-negative information from the set of the signal values by using a pre-stored basis calculation algorithm (e.g., a Ct calculation algorithm). In an embodiment, the set of the signal values may be raw data obtained from the detection device, or may be a signal processed by performing predetermined signal processing such as noise reduction, signal correction, or signal extraction on the raw data.

The contamination assumption unit 310 may calculate the assumed value indicating the contamination possibility of the plate, by using at least one of (a) the distance between the positive reaction wells; (b) the density of the positive reaction wells; and (c) the area or the shape of the figure defined according to the line connecting the positive reaction wells based on the obtained position data; together with the positive-negative determination basis. For example, the contamination assumption unit 310 may determining the distance, the density, the area or the shape as a numerical value by using the position data of the positive reaction wells according to at least one of the first to eighth embodiments described above, and may calculate the assumed value by applying the positive-negative determination basis between the positive reaction wells to the determined numerical value as a weight.

For a more detailed description of this, it is assumed that positive reaction wells are located in the plate as shown in FIG. 17, a first case in which Ct values of the positive reaction well at the coordinate value (1, 1), the coordinate value (2, 1), and the coordinate value (3, 1) are 30, 10, and 37, respectively, and a second case in which the corresponding Ct values are 30, 20, and 25, respectively. As an example, the first case is assumed to be a case in which, as a part of the target analyte included in the true-positive reaction well at the coordinate value (2, 1) splashes on the reaction wells at the coordinate value (3, 1) and the coordinate value (1, 1) nearby due to a contamination source by pipetting, and the reaction wells at the coordinate value (1, 1) and the coordinate value (3, 1) are determined to be false-positive.

In an embodiment, the contamination assumption unit 310 may determine the distance between each positive reaction well and each of the other positive reaction wells, and may determine the assumed value based on a result of applying each weight to reciprocals of each distance between each positive reaction well and other positive reaction wells and summing thereof. For example, when there are three positive reaction wells in the plate such as in the first case or the second case, the Manhattan distance from each positive reaction well to other positive reaction wells may be calculated, and the assumed value may be calculated to be inversely proportional to each Manhattan distance and proportional to each weight.

Here, the weight may be determined based on the positive-negative determination basis between each positive reaction well and other positive reaction wells. In an embodiment, the weight is determined according to the difference in the positive-negative determination basis between each positive reaction well and other positive reaction wells, and the difference may be, for example, a difference in Ct value between each positive reaction well and another positive reaction well. Here, the difference may mean a magnitude difference in each value (e.g. $|A-B|$) or a calculated value (e.g., $(A-B)^2$) based on the magnitude difference.

In an embodiment, the assumed value may be calculated based on Equations 5 and 6 below. In Equation 5 and Equation 6, $a_n$ represents the assumed value for the nth positive reaction well of the plate, $d_m$ represents the maximum distance that can be calculated from the coordinate value (x, y) (e.g., in the case of the plate having a 12 x 8 structure, the maximum Manhattan distance is 18), $d_i$ represents the distance between the nth positive reaction well and another positive reaction well (e.g., in the case of the Manhattan distance, $d_i=i$), and $w_i$ represents the difference in Ct values between the nth positive reaction well and another positive reaction wells when the distance between the nth positive reaction well and another positive reaction well is $d_i=i$. In addition, a represents the assumed value (a) of contamination of the plate, and $n_p$ represents the count of positive reaction wells of the plate.

[Equation 5]

$$a_n = \sum_{i=1}^{d_m} \frac{1}{d_i} \, w_i$$

[Equation 6]

$$a = \sum_{n=1}^{n_p} a_n$$

As a specific example, in the first case, as shown in FIG. 17, when three positive reaction wells are located adjacent to each other and the difference in Ct value between the adjacent positive reaction wells is relatively large, the assumed value $a_1$ of the first positive reaction well at the coordinate value (1, 1) is 23.5, which is a result of summing: (a) 20 (=20*(1/1)), which is a product of multiplying the reciprocal of the distance (=1) from the second positive reaction well at the coordinate value (2, 1) by the difference (=20) in Ct value from the second positive reaction well; and (b) 3.5 (=7*(1/2)), which is a product of multiplying the reciprocal of the distance (=2) from the third positive reaction well at the coordinate value (3, 1) by the difference (=7) in Ct value from the third positive reaction well. In the same manner, the assumed value $a_2$ of the second positive reaction well at coordinate value (2, 1) is 47 (=20*(1/1) + 27*(1/1)), and the assumed value $a_3$ of the third positive reaction well at coordinate value (3, 1) is 30.5 (=27*(1/1) + 7*(1/2)). Accordingly, the assumed value a for contamination of the plate may be calculated as 101, which is the sum of $a_1$ to $a_3$.

As another example, in the second case, as shown in FIG. 17, when three positive reaction wells are located adjacent to each other and the difference in Ct value between the adjacent positive reaction wells is relatively small, the assumed value a for contamination of the plate may be calculated as 35, which is the sum of $a_1$ (=10*(1/1) + 5*(1/2)), $a_2$ (=10*(1/1) + 5*(1/1)), and $a_3$ (=5*(1/1) + 5*(1/2)).

As described above, when the assumed values in the first case and the second case are compared, even if the plurality of positive reaction wells are located adjacent to each other in the plate, the assumed value for contamination of the plate may be differently calculated according to the difference in Ct values between the adjacent positive reaction wells. In particular, such as in the first case, the larger the difference in Ct values between adjacent positive reaction wells, the larger the assumed value of contamination may be calculated.

Meanwhile, according to an embodiment, when the positive-negative determination basis is Ct, the Ct value of a specific reaction well or the difference in the Ct values between two reaction wells may be determined based on a threshold value (cutoff) preset for Ct. For example, in the first case, when the threshold value for Ct is 35, the reaction well at (3, 1) may be processed as a negative reaction well rather than a positive reaction well, and thus may be excluded from the process of obtaining the difference in Ct values between the positive reaction wells.

In another embodiment, the contamination assumption unit 310 may calculate the assumed value based on a result of applying the difference in the positive-negative determination basis between the positive reaction wells as a weight to the density of the positive reaction wells and calculating thereof. For example, the contamination assumption unit 310 may generate the line connecting the positions of other positive reaction wells within a range of a predetermined maximum connection distance (e.g., 2) for the positions of each positive reaction well, and may calculate the assumed value based on a result of multiplying a value calculated from the count of generated lines and the length of the line by the difference in Ct values between the corresponding positive reaction well and other positive reaction wells as a weight.

In another embodiment, the contamination assumption unit 310 may calculate the assumed value based on a result of applying the difference in the positive-negative determination basis between the positive reaction wells as a weight to the area or the shape of the figure. For example, the contamination assumption unit 310 may determine the area of the polygon obtained according to the line connecting the positions of the positive reaction wells, and calculate the assumed value based on a result of multiplying the area of each polygon by the difference in the Ct values between the positive reaction wells included in the polygon as a weight.

In another embodiment, the contamination assumption unit 310 detect the count of positive reaction wells included in each sliding window 20 while moving the sliding window 20 on the image area 10, and calculate the assumed value based on a result of applying the difference in the positive-negative determination basis between the positive reaction wells as a weight to the detected counts.

As described above, according to various embodiments, the assumed value of the contamination of the plate may be calculated by using the position data of the positive reaction wells and the positive-negative determination basis, and according to embodiments, the method for calculating the assumed value may be modified and implemented in various ways based on the technical idea described throughout the specification.

In an embodiment, when the calculated assumed value is not less than a predetermined threshold value, the contamination assumption unit 310 may assume that contamination has occurred in the plate. Alternatively, the contamination assumption unit 310 may assume that contamination has occurred when the ratio criterion between the count of positive reaction wells and the assumed value of contamination in the plate satisfies a preset criterion.

In another embodiment, the contamination assumption unit 310 may calculate the assumed value by using at least one of the distance between the positive reaction wells, the density, the area or the shape of the figure according to the above-described embodiments, and may assume that contamination has occurred in the plate when the assumed value is not less than a predetermined threshold value and the preset criterion is satisfied for the positive-negative determination basis of the reaction wells. For example, even if the assumed value is equal to or greater than the threshold value, criterions may be additionally identified. The criterions may be such as (a) whether the difference in the positive-negative determination basis between each positive reaction well and other positive reaction wells is equal to or greater than a preset value (e.g., 10); or (b) whether the count of pairs of positive reaction wells, in which the difference in the positive-negative determination basis between adjacent positive reaction wells within a predetermined distance in the plate is not less than a preset value, is equal to or greater than a preset count. When the corresponding criterions are satisfied, it may be assumed that contamination has occurred in the plate.

Meanwhile, according to an embodiment, the contamination assumption unit 310 may assume the contamination in unit of positive reaction well by using the position data of the positive reaction wells and the positive-negative determination basis. For example, the contamination assumption unit 310 may calculate the first assumed value in unit of the positive reaction well based on a result of applying each weight to reciprocals of each distance between each positive reaction well and other positive reaction wells and summing thereof. In addition, the contamination assumption unit 310 may assume (a) whether the reaction well the contamination in unit of the positive reaction well has occurred, (b) whether the positive reaction well is a contaminated false-positive reaction well, (c) whether the positive reaction well is a true-positive reaction well that provides a nucleic acid molecule extracted from the pathogen to the contaminated reaction well, or (d) the like, in consideration of the value distribution, the sign pattern, or the like of the first assumed values of the plate.

According to an embodiment, the contamination assumption unit 310 may assume the contamination in unit of plate based on the assumed results in unit of positive reaction well. For example, the contamination assumption unit 310 may calculate the second assumed value for the contamination possibility of the plate by calculating the first assumed values in unit of positive reaction well in a predetermined manner (e.g., summation).

According to an embodiment, the contamination assumption unit 310 may assume the contamination of the plate by using the positive-negative determination basis between the reaction wells, rather than by using the position data of the positive reaction wells together. For example, the contamination assumption unit 310 may analyze a pattern in which the difference in Ct values between the reaction wells is distributed in the plate, and may assume the contamination of the plate according to whether the analyzed pattern matches any one of pre-stored contamination patterns.

According to an embodiment, the contamination assumption unit 310 may apply, as a weight, the difference in the positive-negative determination basis between the positive reaction wells only when a preset criterion for the distance between the positive reaction wells is satisfied in the process of calculating the assumed value. For example, the contamination assumption unit 310 may measure the difference in the positive-negative determination basis between the two positive reaction wells only when they are located adjacent to each other within a preset distance, and may reflect the difference in the process of calculating the assumed value. In addition, the preset distance may be set or updated by the user.

According to an embodiment, the contamination assumption unit 310 may apply, as a weight, the difference in the positive-negative determination basis between the positive reaction wells only when a preset criterion for the range of the positive-negative determination basis is satisfied in the process of calculating the assumed value. For example, the contamination assumption unit 310 may reflect the Ct value difference between the two positive reaction wells in the process of calculating the assumed value only when the Ct value belongs to a preset range (e.g., 10 or more and 35 or less) or when the measured Ct value difference belongs to a preset range (e.g., 10 or more).

As described above, according to the above-described ninth embodiment, it is possible to implement higher assumption accuracy than when the contamination is assumed by only using the position data of the positive reaction wells.

In the above, various embodiments of the function and/or operation of the contamination assumption unit 310 assuming the contamination by using the position data of the positive reaction wells have been described. However, the disclosure is not limited to the above-described embodiment, and may be implemented in various modified forms by applying various public algorithms based on the technical spirit described throughout the specification. For example, a machine learning algorithm, a statistical algorithm, and the like may be used in a process of assuming contamination, and for example, the method may be performed by clustering adjacent positive reaction wells on a plate by using a machine learning-based K-means data clustering algorithm, and then detecting whether the density calculated for each cluster within the clustered range is equal to or greater than a predetermined level.

Hereinafter, various embodiments of a function and/or an operation of providing meaningful information to a user by using the result of assuming the contamination by the contamination warning unit 320 and the quality management unit 330 will be described.

In an embodiment, the contamination warning unit 320 may transmit the result of assuming the contamination of the plate to the display 130, and the display 130 may display the transmitted result. In an embodiment, the result of assuming the contamination of the plate may include at least one of the assumed value for contamination calculated for the plate, the threshold value, and whether the plate is contaminated assumed according to a result of comparing the assumed value and the threshold value. In an embodiment, the result of assuming the contamination of the plate may further include at least one of the first assumed value calculated for the contamination possibility of each of the plurality of positive reaction wells, the second assumed value calculated for the contamination possibility of the plate based on the first assumed values, the first threshold value, the second threshold value, the first graph 40, the second graph 50, and the third graph 60.

In an embodiment, the contamination warning unit 320 may output a warning message indicating that at least in part of the positive reaction wells are likely to be positive due to contamination, when the assumed value indicating the contamination possibility of the plate is not less than the threshold value 30. For example, when the assumed value is greater than the threshold value 30, the contamination warning unit 320 may display the warning message on the display 130 or transmit the warning message to an administrator terminal (not shown) associated with the corresponding plate. In an embodiment, the warning message may include the plate identifier, the assumed value, the threshold value 30, and a message recommending performing a re-detection process for that plate.

As described above, while the prior art requires re-test to check whether contamination has occurred in the plate, an embodiment of the present disclosure may quantitatively assume whether contamination has occurred in the plate by using the position data of the positive reaction well of the plate without such a re-test.

In addition, while the prior art requires re-test of all (or a considerable count of) plates to provide accurate test results, an embodiment of the present disclosure may reduce the count of unnecessary re-tests and shorten the waiting time of the examinee by recommending re-test of a plate of which contamination is assumed. Alternatively, while the prior art results in providing a relatively inaccurate test result as the re-test is omitted for all (or a considerable count of) plates due to an excessive cost problem, an embodiment of the present disclosure may provide a more accurate test result for an examinee by recommending a re-test for a plate of which contamination is assumed.

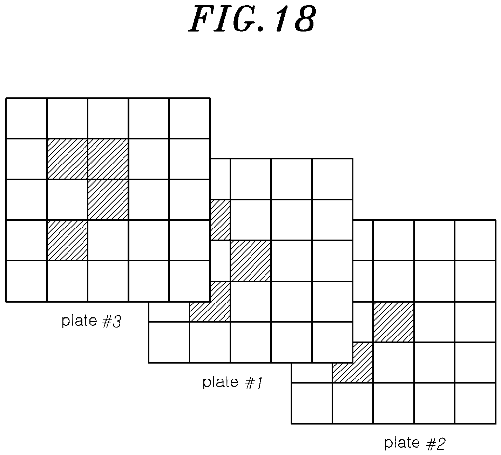

In an embodiment, the contamination warning unit 320 may output a warning message further including the position data of the positive reaction well assumed to be contaminated among the positive reaction wells, based on the plurality of the first assumed values indicating the contamination possibility of each of the plurality of positive reaction wells. For example, when the second assumed value is greater than the second threshold value, the contamination warning unit 320 may transmit (a) the coordinate values of one or more positive reaction wells, which are greater than the first threshold value, among the plurality of the first assumed values, (b) the plate identifier, and (c) the warning message to the administrator terminal (not shown) associated with the corresponding plate. As described above, by providing the position data of the positive reaction well of which contamination is assumed together, it is possible to support a re-test to be performed with a focus on the position of the positive reaction well of which contamination is assumed. Referring to FIG. 18, the contamination warning unit 320 may provide the plate identifier in order from the highest to lowest assumed values among the plurality of plates. For example, the contamination warning unit 320 may arrange the plurality of assumed values calculated for each of the plurality of plates in descending order according to the size, and display the result of arranging the identification numbers of the plates together according to the arranged assumed values on the display 130. For example, the identification number of the plate, the image indicating the position of the positive reaction well in the corresponding plate, and the assumed value may be provided together according to the arranged order.

In an embodiment, the contamination warning unit 320 may arrange and display the plurality of the first assumed values on one plate. In addition, the contamination warning unit 320 may provide the reaction well identifier of the positive reaction well in a descending order of the first assumed value. In addition, the contamination warning unit 320 may provide a highlight mark so that the positive reaction well having the highest first assumed value is visually distinguished from other positive reaction wells, and may also provide a graphic interface that allows intuitive indication of the magnitude of the second assumed value through a gradual display of a specific color (e.g., red) indicating that contamination is assumed.

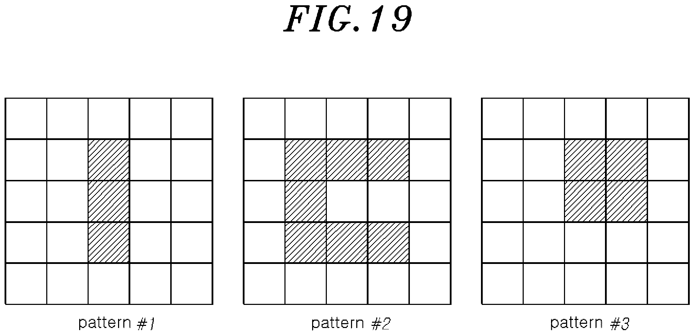

Referring to FIG. 19, the contamination warning unit 320 may provide assumption information about a contamination pattern of one or more positive reaction wells of the plate based on the position data of the positive reaction well. In an embodiment, the contamination warning unit 320 may analyze whether a line connecting the positive reaction wells or a figure defined according to the line is the same as or similar to any one of a plurality of pre-stored contamination patterns, and may generate assumption the information about the contamination pattern according to the analysis result. For example, when the line connecting the positive reaction wells is a straight line, has a "C" shape, or has a square shape without a negative reaction well, the contamination warning unit 320 may determine that the line corresponds to any one of the plurality of pre-stored contamination patterns, and may output a message indicating that the positive reaction wells are assumed to have the corresponding contamination pattern in the plate. In an embodiment, the identification number of the plate, the image indicating the position of the positive reaction well in the plate, and information about the assumed contamination pattern (e.g., a pattern image, a description about the situations under which the corresponding contamination pattern occurs, etc.) may be provided together. According to an embodiment, the assumption information about the contamination pattern may further include information about the contamination source, and for example, may further include assumption information on which one of the first contamination source to the third contamination source the contamination assumed to have occurred in the plate is caused by.

In an embodiment, a machine learning algorithm may be used in the process of providing the assumption information about the contamination pattern. For example, a machine learning-based classification model may be obtained by using a training dataset including a plurality of images for the positions of positive reaction wells classified into a plurality of contamination patterns. In addition, as the image area 10 of the plate, which is the assumed target of contamination, is input to the classification model, the presence or absence of the contamination pattern, the classification value of the contamination pattern, and the like may be obtained.

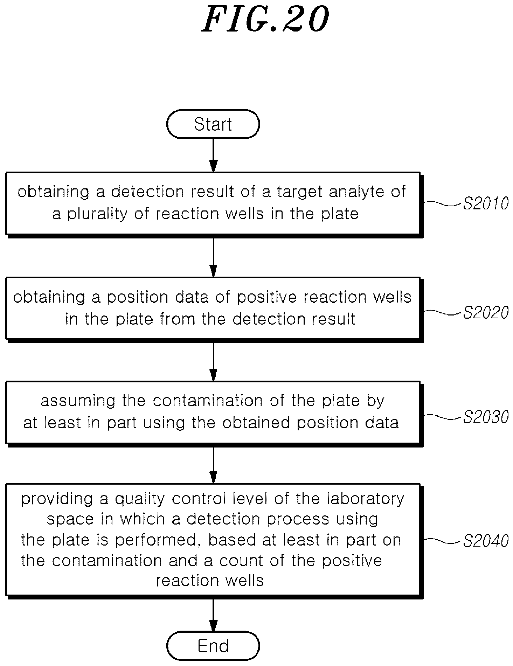

Referring to FIG. 20, the quality management unit 330 may perform a method for managing quality of a laboratory space. Steps S2010 to S2030 may be performed by the components of the computer device 100 according to the above-described embodiment.

In step S2040, the quality management unit 330 may provide a quality control level of the laboratory space in which a detection process using the plate is performed, based at least in part on the contamination of the plate and the count of the positive reaction wells, which are assumed by the contamination assumption unit 310. In an embodiment, the quality management unit 330 may determine the quality control level of the laboratory space based on the ratio between the count of positive reaction wells and the assumed value of the contamination.

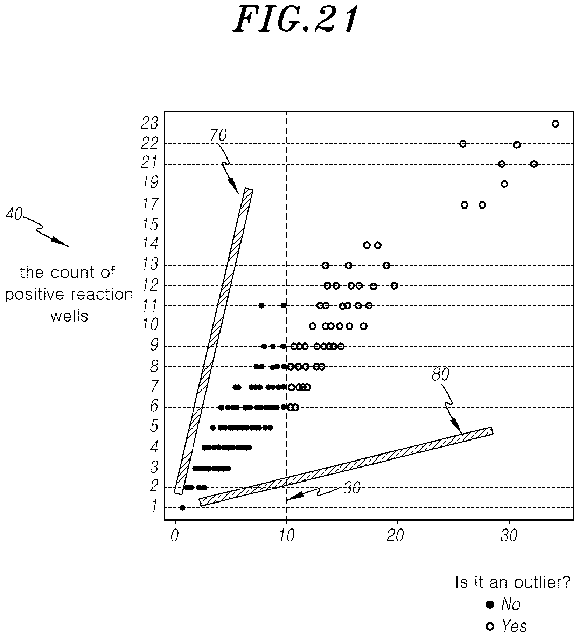

Referring to FIG. 21, as described above, the first graph 40 illustrates the change in the count of positive reaction wells according to the change in the assumed value of the contamination, and the assumed value of the contamination and the count of positive reaction wells are relatively proportional to each other within a predetermined range.

The quality management unit 330 may collect a plurality of detection results for a plurality of plates obtained in a specific laboratory space, and may store and manage a plurality of assumed values obtained from the plurality of collected detection results and the count of a plurality of positive reaction wells. The quality management unit 330 may obtain the first graph 40 in which the coordinate values (X, Y), in which the assumed value of the contamination and the count of positive reaction wells are paired, are displayed in a two-dimensional coordinate system, for each of the plurality of detection results.

The quality management unit 330 may assume whether the quality management is well performed in the corresponding laboratory space based on the ratio between the assumed value of the contamination and the count of positive reaction wells shown in the first graph 40.

In an embodiment, when the quality control level is assumed using the plurality of detection results, the quality management unit 330 may display, in the first graph 40, the coordinate value (X, Y) in which the assumed value of contamination obtained from the plurality of detection results in the corresponding laboratory space and the count of positive reaction wells are paired, and may assume whether the quality management is well performed in the corresponding laboratory space by comparing a slope shown in the first graph 40 with a preset value. In another embodiment, when the quality control level is assumed using one detection result, the quality management unit 330 may calculate a ratio of the count of the positive reaction well to the assumed value calculated for the corresponding plate, and assume whether the quality control is well performed in the corresponding laboratory space by comparing the calculated ratio with a preset value.

The quality management unit 330 may output a first message indicating that a positive state for the quality control level is assumed, when the ratio of the count of positive reaction wells to the assumed value indicating the contamination possibility of the plate is not less than a first value. For example, the quality management unit 330 may determine a slope based on a line connecting Y coordinate values located at the uppermost end or the upper end in the first graph 40, and may output the first message when the determined slope is equal to or greater than a first slope 70 corresponding to a first value (e.g., a slope a value when expressed by a two-dimensional function y=ax+b).

For example, when the determined slope is equal to or greater than the first slope 70, it may correspond to a case where the count of positive reaction wells per plate is relatively large compared to the assumed value. In other words, even though there are many positive reaction wells, the contamination possibility may be considered relatively low because there are many plates having a relatively long distance between the positive reaction wells in the plates and the assumed values are relatively small.

In an embodiment, the first message may further include a message recommending to skip performing a re-detection process for some plates in which the ratio of the count of positive reaction wells to the assumed value is not less than the first value, among a plurality of plates. For example, even though there are many positive reaction wells, a message may be provided indicating that retest for the corresponding positive reaction well may be skipped because the assumed value is relatively small compared to the count of positive reaction wells. Accordingly, the count of unnecessary retests may be reduced, and the waiting time of the examinee may be reduced, thereby improving operation efficiency throughout the molecular diagnostic test process.

The quality management unit 330 may output a second message indicating that a negative state for the quality control level is assumed, when the ratio of the count of positive reaction wells to the assumed value is not larger than a second value. Here, the second value may be less than the first value. For example, the quality management unit 330 may determine a slope based on a line connecting Y coordinate values located at the lowermost end or the lower end in the first graph 40, and may output the second message when the determined slope is less than or equal to a second slope 80 corresponding to a second value (e.g., a slope c value when expressed by a two-dimensional function y=cx+d).

For example, when the determined slope is less than or equal to the second slope 80, it may correspond to a case where the count of positive reaction wells per plate is relatively small compared to the assumed value. In other words, even though there are few positive reaction wells, the contamination possibility may be considered relatively high because there are many plates having a relatively narrow distance between the positive reaction wells and the assumed values are relatively large. In this respect, it may be assumed that the corresponding laboratory space is not well managed for quality.

In one embodiment, the second message may further include a message recommending at least one of (a) cleaning the laboratory space, (b) replacing a pipette, and (c) re-performing the detection process for some plates in which the ratio of the count of positive reaction wells to the assumed value is not larger than the second value, among the plurality of plates. For example, when the negative state for the quality control level is assumed, a message recommending that the contamination source be removed by cleaning the laboratory space or replacing the experimental device such as pipettes, or a message recommending that the detection process be performed again since there is a possibility of the false-positive due to contamination with respect to one or more plates of which the assumed value is calculated to be smaller than the second value among the plurality of plates of which the detection result is previously obtained in the corresponding laboratory space. Accordingly, when the contamination is assumed, it is possible to reduce the social cost involved according to the false-positive by recommending re-test, and improve the quality management of the laboratory space.

In an embodiment, the first and second messages may further include at least one of an identifier of the laboratory space, the determined slope, the first slope 70, and the quality control level. In addition, the quality control level may be calculated as a numerical value based on a difference between the determined slope and the first slope 70.

According to an embodiment of the present disclosure, the degree to which the positive reaction wells are adjacent to each other in the plate may be calculated as a quantitative assumed value and used as an index indicating the possibility that contamination has occurred in the corresponding plate.

According to an embodiment of the present disclosure, the contamination possibility in unit of positive reaction well may be assumed, and the position data of the positive reaction well where contamination is assumed to be occurred may be provided.

According to an embodiment of the present disclosure, by setting the threshold value 30 which is a comparison criterion of the assumed value based on a predetermined dataset, the presence of contamination may be effectively assumed by checking whether the assumed value corresponds to a statistical outlier.

According to an embodiment of the present disclosure, when it is assumed that contamination has occurred, a warning message may be provided indicating that at least some of the one or more positive reaction wells of the plate are likely to be positive due to contamination, thereby effectively informing the user of the contamination possibility.

According to an embodiment of the present disclosure, meaningful information on whether quality management is well performed in the corresponding laboratory space may be provided by using the assumed value of contamination and the count of positive reaction wells.

Combinations of each block of the block diagram attached to the present invention and each step of the flowchart may be performed by computer program instructions. Since these computer program instructions may be mounted on an encoding processor of a general-purpose computer, special-purpose computer, or other programmable data processing device, the instructions executed by the encoding processor of the computer or other programmable data processing device generate means for performing the functions described in each block of the block diagram or each step of the flowchart. Since the computer program instructions can also be stored in a computer-usable or computer-readable memory that can be directed to a computer or other programmable data processing equipment to implement functions in a particular method, the instructions stored in the computer-usable or computer-readable memory may produce an item of manufacture including instruction means for performing the functions described in each block of the block diagram or each step of the flowchart. Since the computer program instructions can also be mounted on a computer or other programmable data processing device, the instructions that perform a series of operation steps on the computer or other programmable data processing device to generate a computer-executable process, thereby performing the computer or other programmable data processing device, can also provide steps for executing the functions described in each block of the block diagram and each step of the flowchart.

In addition, each block or each step may represent a module, a segment, or a portion of code including one or more executable instructions for executing the specified logical function(s). It should also be noted that in some alternative embodiments, functions mentioned in the blocks or steps may occur out of order. For example, two blocks or steps shown in succession may in fact be performed substantially simultaneously or the blocks or steps may sometimes be performed in reverse order depending on the corresponding function.

The above description is merely illustrative of the technical idea of the present disclosure, and those skilled in the art to which the present disclosure pertains will be able to make various modifications and variations without departing from the essential quality of the present disclosure. Accordingly, the embodiments disclosed in the present disclosure are not intended to limit the technical idea of the present disclosure, but to explain it, and the scope of the technical idea of the present disclosure is not limited by these embodiments. The protection scope of the present invention should be interpreted by the following claims, and all technical ideas within the scope equivalent thereto should be interpreted as being included in the scope of the present invention.

## Claims

1. A method for assuming a contamination of a plate used for a nucleic acid amplification test, the method being performed by a computer device, the method comprising:

   obtaining a detection result of a target analyte of a plurality of reaction wells in the plate;
   obtaining a position data of positive reaction wells in the plate from the detection result; and
   assuming the contamination of the plate by at least partially using the obtained position data.

2. The method of claim 1, wherein the contamination comprises a false-positive occurrence in at least some of the positive reaction wells due to one or more contamination sources, and
   wherein the one or more contamination sources comprise at least one selected from the group consisting of: a first contamination source caused by a nucleic acid molecule extracted from a pathogen in a positive reaction well being propagated into the air; a second contamination source caused by the nucleic acid molecule of a pipette in contact with the positive reaction well; a third contamination source caused by a poor sealing of the reaction well, and a combination thereof.

3. The method of claim 1, wherein the assuming the contamination comprises:

   determining at least one of (a) a distance between the positive reaction wells, (b) a density of the positive reaction wells, and (c) an area or a shape of a figure defined according to a line connecting the positive reaction wells based on the obtained position data; and
   assuming the contamination by using at least one of: the distance; the density; and the area or the shape of the figure.

4. The method of claim 1, further comprising obtaining an image area for the position of the plurality of reaction wells based on the position data,
   wherein the assuming the contamination comprises:

   detecting a count of positive reaction wells included in each sliding window while moving a sliding window of a preset size by a preset unit on the image area; and
   calculating an assumed value for the contamination based on the count of positive reaction wells included in each sliding window.

5. The method of claim 4, wherein the detecting the contamination is performed by using the sliding window of the preset size and then performed by using each of sliding windows of a size different from the preset size, and
   the assumed value is calculated based on the count of positive reaction wells included in each sliding window detected by using the sliding window of the preset size and the sliding window of the size different from the preset size.

6. The method of claim 4, wherein the moving the sliding window comprises:

   moving the sliding window on the image area by a preset unit smaller than the preset size; or
   moving the sliding window on the image area so that a part of the positive reaction wells included in the sliding window before moving is included in the sliding window after moving.

7.   The method of claim 4, wherein the calculating the assumed value comprises:

detecting a frequency for each count of positive reaction wells in the sliding window; and
calculating a degree to which the positive reaction wells are adjacent to each other in the plate, as the assumed value, by using each count of the positive reaction wells in the sliding window, the size, and the frequency.

8.   The method of claim 1, wherein the assuming the contamination comprises assuming a contamination in each of the positive reaction wells by using at least a part of the obtained position data.

9.   The method of claim 8, wherein the assuming the contamination comprises assuming the contamination of the plate by at least partially using the assumed result of the contamination in each of the positive reaction wells.

10.  The method of claim 8, wherein the assuming the contamination in each of the positive reaction wells comprises:

determining a distance between the positive reaction wells by using the obtained position data; and
calculating each of a first assumed values indicating a contamination possibility in each of the positive reaction wells by using the distance between the positive reaction wells.

11.  The method of claim 10, wherein the distance between the positive reaction wells is calculated based on a Manhattan distance measurement method.

12.  The method of claim 10, wherein the distance is a distance between each positive reaction well and other positive reaction wells, and
wherein the each of the first assumed values is determined by at least partially using a result of summing reciprocals of the distance between each positive reaction well and other positive reaction wells.

13.  The method of claim 10, wherein the assuming the contamination further comprises calculating a second assumed value indicating a contamination possibility of the plate by using a result of summing the first assumed values.

14.  The method of claim 1, further comprising displaying a result of the assuming the contamination.

15.  The method of claim 1, further comprising outputting a warning message indicating that at least in part of the positive reaction wells are likely to be positive due to contamination, when an assumed value indicating the contamination possibility of the plate is not less than a predetermined threshold value.

16.  The method of claim 15, wherein the warning message further comprises a position data of a positive reaction well assumed to be contaminated among the positive reaction wells, and wherein the positive reaction well assumed to be contaminated is determined by using an assumed value indicating a contamination probability in each of the positive reaction wells.

17.  The method of claim 15, wherein the threshold value is determined based on an interquartile range (IQR), a third quartile (Q3), and preset weight which are determined from a dataset for the assumed values of a plurality of plates.

18.  The method of claim 1, an assumed value indicating a contamination possibility of the plate is calculated for each of a plurality of plates, and
wherein the method further comprises aligning and displaying the assumed value of each of the plurality of plates.

19.  The method of claim 1, further comprising providing an assumption information about a contamination pattern of the positive reaction wells by using the position data of the positive reaction wells.

20.  The method of claim 1, further comprising:

obtaining a re-detection result of the target analyte in the positive reaction wells; and
updating an equation used to assume the contamination and/or a threshold value which is a criterion for assuming the contamination, by using a comparison result between the re-detection result and the detection result.

21. The method of claim 20, when any one positive reaction well among the positive reaction wells is determined to be positive in the detection result but is determined to be negative in the re-detection result, the updating is performed based on a difference of assumed values of the contamination calculated from each of the re-detection result and the detection result.

22. The method of claim 1, wherein the assuming the contamination comprises assuming the contamination of the plate by additionally using a positive-negative determination basis obtained from the detection result.

23. The method of claim 22, wherein the positive-negative determination basis comprises at least one selected from the group consisting of (a) a cycle value indicating a time or reaction number for an intensity of a signal value to reach a predetermined threshold value in a set of signal values obtained from the detection result, (b) a signal value at a specific cycle in the set of the signal values, and (c) a combination thereof.

24. The method of claim 22, wherein the assuming the contamination comprises:

   determining at least one of (a) a distance between the positive reaction wells, (b) a density of the positive reaction wells, and (c) an area or a shape of a figure defined according to a line connecting the positive reaction wells based on the obtained position data; and
   calculating an assumed value indicating a contamination possibility of the plate, by using (i) at least one of the distance, the density, the area or the shape of the figure, and (ii) the positive-negative determination basis between the positive reaction wells.

25. The method of claim 24, wherein the assumed value is calculated based on a result of applying each weight to reciprocals of each distance between each positive reaction well and other positive reaction wells and summing thereof, and
   wherein the weight is determined based on the positive-negative determination basis between each positive reaction well and other positive reaction wells.

26. A computer program stored on a computer-readable recording medium, wherein the computer program is programmed to perform each step included in the method of claim 1.

27. A computer-readable recording medium on which a computer program is stored, wherein the computer program is programmed to perform each step included in the method of claim 1.

28. A computer device comprising:

   a memory comprising at least one instruction; and
   a processor, and
   as the at least one instruction is executed by the processor:

   a detection result of a target analyte of a plurality of reaction wells in the plate is obtained;
   a position data of positive reaction wells in the plate is obtained from the detection result; and
   the contamination of the plate is assumed by at least partially using the obtained position data.

29. A method for managing quality of a laboratory space, the method being performed by a computer device, the method comprising:

   obtaining a detection result of a target analyte of a plurality of reaction wells in the plate;
   obtaining a position data of positive reaction wells in the plate from the detection result; and
   assuming the contamination of the plate by at least in part using the obtained position data; and
   providing a quality control level of the laboratory space in which a detection process using the plate is performed, based at least in part on the contamination and a count of the positive reaction wells.

30. The method of claim 29, wherein the providing the quality control level of the laboratory space comprises:

   outputting a first message indicating that a positive state for the quality control level is assumed, when a ratio of the count of positive reaction wells to an assumed value indicating a contamination possibility of the plate is not less than a first value; and

outputting a second message indicating that a negative state for the quality control level is assumed, when the ratio of the count of positive reaction wells to the assumed value is not larger than a second value, which is less than the first value.

31. The method of claim 30, wherein the first message further comprises a message recommending to skip performing a re-detection process for some plates in which the ratio of the count of positive reaction wells to the assumed value is not less than the first value, among a plurality of plates.

32. The method of claim 30, wherein the second message further comprises a message recommending at least one of (a) cleaning the laboratory space, (b) replacing a pipette, and (c) re-performing the detection process for some plates in which the ratio of the count of positive reaction wells to the assumed value is not larger than the second value, among the plurality of plates.

# FIG.1

1000

100

computer
device

200

detection result
providing device

# FIG.2

100

computer device

110 — obtaining unit

120 — memory

130 — display

processor

140

# FIG.3

310 — contamination assumption unit

320 — contamination warning unit

330 — quality management unit

# FIG.4

```
Start
```

obtaining a detection result of a target analyte
of a plurality of reaction wells in the plate  — S410

obtaining a position data of positive reaction
wells in the plate from the detection result  — S420

assuming the contamination of the plate by at
least partially using the obtained position data  — S430

```
End
```

# FIG.5

# *FIG.6*

| | | | | positive reaction well |
| | | | | negative reaction well |

# FIG.7

positive reaction well

negative reaction well

# FIG.8

|     | $X_1$ | $X_2$ | $X_3$ | $X_4$ | $X_5$ | $X_6$ | $X_7$ | $X_8$ | $X_9$ | $X_{10}$ | $X_{11}$ | $X_{12}$ |
|-----|-------|-------|-------|-------|-------|-------|-------|-------|-------|----------|----------|----------|

$Y_1$
$Y_2$
$Y_3$
$Y_4$
$Y_5$
$Y_6$
$Y_7$
$Y_8$

positive reaction well

negative reaction well

# FIG.9

```
Start
```

obtaining an image area for the position of the
plurality of reaction wells based on the position
data of the positive reaction wells

~ S910

detecting a count of positive reaction wells
included in each sliding window while moving a
sliding window of a preset size by a preset unit
on the image area

~ S920

calculating an assumed value for the
contamination based on the detected count of
positive reaction wells

~ S930

```
End
```

# FIG.10

positive reaction well

negative reaction well

# FIG.11

positive reaction well

negative reaction well

## FIG.12

# FIG.13

<u>50</u>

# FIG.14

Start

assuming a contamination in each of a plurality of positive reaction wells by using the position data of positive reaction wells ~S1410

assuming the contamination of the plate by using the assumed result of the contamination in each of the plurality of positive reaction wells ~S1420

End

# FIG.15

| | | | | | | | | | | | |
|---|---|---|---|---|---|---|---|---|---|---|---|
| (1,1) | (1,2) | (1,3) | (1,4) | (1,5) | (1,6) | (1,7) | (1,8) | (1,9) | (1,10) | (1,11) | (1,12) |
| (2,1) | (2,2) | (2,3) | (2,4) | (2,5) | (2,6) | (2,7) | (2,8) | (2,9) | (2,10) | (2,11) | (2,12) |
| (3,1) | (3,2) | (3,3) | (3,4) | (3,5) | (3,6) | (3,7) | (3,8) | (3,9) | (3,10) | (3,11) | (3,12) |
| (4,1) | (4,2) | (4,3) | (4,4) | (4,5) | (4,6) | (4,7) | (4,8) | (4,9) | (4,10) | (4,11) | (4,12) |
| (5,1) | (5,2) | (5,3) | (5,4) | (5,5) | (5,6) | (5,7) | (5,8) | (5,9) | (5,10) | (5,11) | (5,12) |
| (6,1) | (6,2) | (6,3) | (6,4) | (6,5) | (6,6) | (6,7) | (6,8) | (6,9) | (6,10) | (6,11) | (6,12) |
| (7,1) | (7,2) | (7,3) | (7,4) | (7,5) | (7,6) | (7,7) | (7,8) | (7,9) | (7,10) | (7,11) | (7,12) |
| (8,1) | (8,2) | (8,3) | (8,4) | (8,5) | (8,6) | (8,7) | (8,8) | (8,9) | (8,10) | (8,11) | (8,12) |

positive reaction well

negative reaction well

*FIG.16*

# FIG.17

| (1,1) | (1,2) | (1,3) | (1,4) | (1,5) | (1,6) | (1,7) | (1,8) | (1,9) | (1,10) | (1,11) | (1,12) |
|---|---|---|---|---|---|---|---|---|---|---|---|
| (2,1) | (2,2) | (2,3) | (2,4) | (2,5) | (2,6) | (2,7) | (2,8) | (2,9) | (2,10) | (2,11) | (2,12) |
| (3,1) | (3,2) | (3,3) | (3,4) | (3,5) | (3,6) | (3,7) | (3,8) | (3,9) | (3,10) | (3,11) | (3,12) |
| (4,1) | (4,2) | (4,3) | (4,4) | (4,5) | (4,6) | (4,7) | (4,8) | (4,9) | (4,10) | (4,11) | (4,12) |
| (5,1) | (5,2) | (5,3) | (5,4) | (5,5) | (5,6) | (5,7) | (5,8) | (5,9) | (5,10) | (5,11) | (5,12) |
| (6,1) | (6,2) | (6,3) | (6,4) | (6,5) | (6,6) | (6,7) | (6,8) | (6,9) | (6,10) | (6,11) | (6,12) |
| (7,1) | (7,2) | (7,3) | (7,4) | (7,5) | (7,6) | (7,7) | (7,8) | (7,9) | (7,10) | (7,11) | (7,12) |
| (8,1) | (8,2) | (8,3) | (8,4) | (8,5) | (8,6) | (8,7) | (8,8) | (8,9) | (8,10) | (8,11) | (8,12) |

positive reaction well

negative reaction well

# FIG.18

plate #3

plate #1

plate #2

FIG.19

pattern #1

pattern #2

pattern #3

# FIG.20

```
          ┌─────────────┐
          │    Start     │
          └──────┬──────┘
                 │
                 ▼
   ┌────────────────────────────────────────┐
   │ obtaining a detection result of a target analyte of │────── S2010
   │    a plurality of reaction wells in the plate        │
   └────────────────────┬───────────────────┘
                        │
                        ▼
   ┌────────────────────────────────────────┐
   │ obtaining a position data of positive reaction wells │────── S2020
   │     in the plate from the detection result           │
   └────────────────────┬───────────────────┘
                        │
                        ▼
   ┌────────────────────────────────────────┐
   │     assuming the contamination of the plate by       │────── S2030
   │  at least in part using the obtained position data    │
   └────────────────────┬───────────────────┘
                        │
                        ▼
   ┌────────────────────────────────────────┐
   │ providing a quality control level of the laboratory  │
   │      space in which a detection process using         │────── S2040
   │  the plate is performed, based at least in part on    │
   │   the contamination and a count of the positive       │
   │                  reaction wells                       │
   └────────────────────┬───────────────────┘
                        │
                        ▼
          ┌─────────────┐
          │     End      │
          └─────────────┘
```

# FIG.21

## INTERNATIONAL SEARCH REPORT

| International application No. |
| --- |
| **PCT/KR2023/005092** |

| A. | CLASSIFICATION OF SUBJECT MATTER |
| --- | --- |

**G01N 21/15**(2006.01)i; **B01L 3/00**(2006.01)i; **G06T 7/70**(2017.01)i; **G06T 7/11**(2017.01)i; **G08B 21/18**(2006.01)i; **G06Q 50/10**(2012.01)i

According to International Patent Classification (IPC) or to both national classification and IPC

| B. | FIELDS SEARCHED |
| --- | --- |

Minimum documentation searched (classification system followed by classification symbols)

G01N 21/15(2006.01); C12Q 1/6848(2018.01); C12Q 1/70(2006.01); G01N 35/00(2006.01); G06T 7/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 분석물(analyte), 오염(contamination), 위치(location), 양성(positive) 및 핵산 (nucleic acid)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | US 2014-0307931 A1 (MASSACHUSETTS INSTITUTE OF TECHNOLOGY) 16 October 2014 (2014-10-16)<br> See paragraphs [0038]-[0087] and figures 1-9. | 1-3,8-14,18, 19,22,24-28 |
| Y | | 4-7,15-17,20, 21,23,29-32 |
| Y | US 2005-0273268 A1 (GHOSH, Jayati et al.) 08 December 2005 (2005-12-08)<br> See paragraphs [0041], [0045] and [0055]-[0058] and figures 14-16B. | 4-7 |
| Y | WO 2018-047544 A1 (HITACHI HIGH-TECHNOLOGIES CORPORATION) 15 March 2018 (2018-03-15)<br> See paragraphs [0001]-[0004] and [0043]-[0046] and figures 6-7. | 15-17,20,21,29-32 |
| Y | US 2003-0157523 A1 (FRANTZ, Gretchen et al.) 21 August 2003 (2003-08-21)<br> See paragraphs [0102] and [0187] and figure 7. | 17 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **26 July 2023** | **26 July 2023** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2023/005092**

C.    DOCUMENTS CONSIDERED TO BE RELEVANT

| Category* | Citation of document, with indication, where appropriate, of the relevant passages | Relevant to claim No. |
|---|---|---|
| Y | US 2019-0352705 A1 (CALIFORNIA INSTITUTE OF TECHNOLOGY) 21 November 2019 (2019-11-21)<br>See paragraphs [0106], [0127] and [0181] and figures 5A and 7-8. | 23 |

Form PCT/ISA/210 (second sheet) (July 2022)

| Patent document cited in search report | | | Publication date (day/month/year) | Patent family member(s) | | | Publication date (day/month/year) |
|---|---|---|---|---|---|---|---|
| US | 2014-0307931 | A1 | 16 October 2014 | WO | 2014-172345 | A2 | 23 October 2014 |
| | | | | WO | 2014-172345 | A3 | 12 November 2015 |
| US | 2005-0273268 | A1 | 08 December 2005 | US | 7302348 | B2 | 27 November 2007 |
| WO | 2018-047544 | A1 | 15 March 2018 | CN | 109642910 | A | 16 April 2019 |
| | | | | CN | 109642910 | B | 29 April 2022 |
| | | | | EP | 3511719 | A1 | 17 July 2019 |
| | | | | JP | 6666628 | B2 | 18 March 2020 |
| | | | | US | 11519924 | B2 | 06 December 2022 |
| | | | | US | 2019-0204346 | A1 | 04 July 2019 |
| US | 2003-0157523 | A1 | 21 August 2003 | EP | 1451358 | A2 | 01 September 2004 |
| | | | | JP | 2005-509870 | A | 14 April 2005 |
| | | | | WO | 03-044213 | A2 | 30 May 2003 |
| | | | | WO | 03-044213 | A3 | 28 August 2003 |
| US | 2019-0352705 | A1 | 21 November 2019 | EP | 3058100 | A2 | 24 August 2016 |
| | | | | US | 10196684 | B2 | 05 February 2019 |
| | | | | US | 11434526 | B2 | 06 September 2022 |
| | | | | US | 2017-0152553 | A1 | 01 June 2017 |
| | | | | WO | 2015-058008 | A2 | 23 April 2015 |
| | | | | WO | 2015-058008 | A3 | 25 June 2015 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4683195 A, Mullis
- US 4683202 A
- US 4800159 A

**Non-patent literature cited in the description**

- **SAIKI et al.** *Science*, 1985, vol. 230, 1350-1354
- **SAMBROOK, J. et al.** Molecular Cloning. A Laboratory Manual. Cold Spring Harbor Press, 2001